# EUROPEAN PATENT APPLICATION

(11) **EP 4 397 274 A1**
(43) Date of publication of application: **10.07.2024**
(21) Application number: 22864424.1
(22) Date of filing: 26.08.2022
(51) Int. Cl.: A61F 2/02, A61L 27/34, A61L 27/40

(54) **TISSUE BODY FORMATION DEVICE AND TISSUE BODY FORMATION METHOD**

(30) Priority: 31.08.2021 JP 2021141332
(71) Applicant: Biotube Co., Ltd., Tokyo, 104-0033 (JP)
(72) Inventor: NAKAYAMA Yasuhide, Tokyo 104-0033 (JP)
(74) Representative: Westphal, Mussgnug & Partner Patentanwälte mbB
(86) International application number: PCT/JP2022/032189
(87) International publication number: WO 2023/032840

(57) **Abstract**

Provided are a tissue body formation device that enables an improvement in the ratio of a dense connective tissue, and a tissue body formation method. An exterior part 10 that separates an environment and a hollow space in a partition wall is provided with a plurality of through holes 20H and 30H leading from the environment to the hollow space. The opening size of the through holes 20H and 30H is 0.02-2.5 mm inclusive. In the connective tissue body, the part requiring a dense connective tissue is a dense part. The surface of the partition wall in contact with the dense part is composed of a non-degradable resin. The compression strength of the non-degradable resin is 10-60 MPa inclusive and the water contact angle of the non-degradable resin is 90°or more.

## Description

### TECHNICAL FIELD

The present invention relates to a tissue body formation device for forming a connective tissue body, and a tissue body formation method using the tissue body formation device.

### BACKGROUND ART

The body's self-defense function encapsulates foreign matter with a capsule mainly composed of fibroblasts and collagen. One type of regenerative medicine, which is a medical treatment that regenerates lost tissues or organs, implants a tissue body formation device as foreign matter in a living body, causing the self-defense function to form a biogenic connective tissue body from living cells in the implanted tissue body formation device (see Patent Documents 1 to 4, for example).

The first example of a tissue body formation device includes two tissue body formation surfaces facing each other. A living tissue material enters the space between these tissue body formation surfaces to form a connective tissue body (see Patent Documents 5 and 6, for example). The second example of a tissue body formation device includes openings with an occupancy rate of 20% or greater and 40% or less in the outer surface of the connective tissue formation device to shorten the implantation period of the tissue body formation device and improve the structural precision of the connective tissue body (see Patent Document 7, for example). The third example of a tissue body formation device includes an opening in the outer surface of the connective tissue formation device to form fibrous connective tissue in the shape of a recess conforming to the opening. The device causes somatic stem cells to accumulate in the recess formed by the fibrous connective tissue (see Patent Document 8, for example).

### CITATION LIST

### PATENT LITERATURE

Patent Document 1: Japanese Laid-Open Patent Publication No. 2007-37763
Patent Document 2: Japanese Laid-Open Patent Publication No. 2007-312821
Patent Document 3: Japanese Laid-Open Patent Publication No. 2008-237896
Patent Document 4: Japanese Laid-Open Patent Publication No. 2010-094476
Patent Document 5: Japanese Laid-Open Patent Publication No. 2014-030598
Patent Document 6: Japanese Laid-Open Patent Publication No. 2017-169778
Patent Document 7: Japanese Laid-Open Patent Publication No. 2017-202180
Patent Document 8: Japanese Patent No. 6813923

### SUMMARY OF INVENTION

### TECHNICAL PROBLEM

The connective tissue body formation technique described above achieves (i) shortening the period for forming a connective tissue body, and (ii) forming the connective tissue body into a desired shape. Forming a connective tissue body into a desired shape in a short period while reducing the burden on the biological environment is required in the stage for demonstrating the possibility that connective tissue body formation technique can contribute to regenerative medicine, in other words, in the stage of basic research.

In recent years, the connective tissue body formation technique has reached the stage of verifying safety and effectiveness as regenerative medicine, in other words, the stage of clinical research. One of the new challenges that this stage of clinical research poses to the connective tissue body formation technique is (iii) converting the connective tissue bodies themselves into strong structures. For example, a connective tissue body with a strong structure has a relatively high density and thus limits penetration of blood into the connective tissue body. It is therefore sufficiently conceivable that the connective tissue body limits thrombus formation.

On the other hand, the connective tissue body formed by the tissue body formation device includes the following two types of connective tissue in separate sections (see Patent Document 8, for example).
(a) Loose connective tissue with low fiber density
(b) Dense connective tissue with high fiber density

In loose connective tissue, which contains more type III collagen than type I collagen, collagen fibers are loosely arranged such that the longitudinal directions of the collagen fibers are disordered. In contrast, in dense connective tissue, which contains more type I collagen than type III collagen, collagen fibers are arranged closely such that the longitudinal directions of the collagen fibers are aligned. When the proportion of such dense connective tissue is increased in the connective tissue body, (iii) the connective tissue body can have a stronger structure. As such, as the connective tissue body formation technique described above, there has been a need for a new technique for increasing the proportion of dense connective tissue in a desired section within the space defined by the tissue body formation device.

### SOLUTION TO PROBLEM

A tissue body formation device for solving the above problems is a tissue body formation device for forming a connective tissue body in an environment in which a living tissue material is present. The tissue body formation device includes a partition wall that defines a hollow space for forming the connective tissue body from the living tissue material. An exterior portion of the partition wall that separates the environment from the hollow space includes through-holes that extend between the environment and the hollow space, the through-holes having an opening size of 0.02 mm or greater and 2.5 mm or less. A section of the connective tissue body that includes dense connective tissue is a dense section. A surface of the partition wall that is in contact with the dense section includes non-degradable resin. The non-degradable resin has a compressive strength of 10 MPa or greater and 60 MPa or less, and the non-degradable resin has a contact angle with water of 90° or greater.

The surface of a device such as an artificial joint that is intended to bond the device to the biological environment is typically required to have properties that limit encapsulation of the device. The initial reaction of encapsulation is believed to be the adsorption of plasma proteins to the surface of the device. Based on this point of view, the surface of a device intended for bonding the device to the biological environment may be modified with water-soluble polymer chains, for example, to limit the adsorption of plasma proteins.

In contrast, a tissue body formation device that is implanted in a biological environment, which is an environment in which a living tissue material is present, only for a predetermined time requires to promote collagen production similar to the encapsulation process in the hollow space of the tissue body formation device. Plasma proteins adsorbed to the surface of the tissue body formation device are considered to induce the progress of biological reactions required for connective tissue body formation, such as adsorption of fibroblasts to plasma proteins and expression of propeptide cleaving enzymes. From this point of view, the surface of the partition wall that defines the hollow space has conventionally been set to have a contact angle with water of 60°or greater and 80°or less, which is the range that most promotes adsorption of plasma proteins.

On the other hand, the hollow space in which the tissue body formation device forms a connective tissue body is not a space in contact with the outer surface of the partition wall, but a space in contact with the inner surface of the partition wall. The formation of a connective tissue body within the hollow space may require adsorption of plasma proteins to the inner surface of the partition wall rather than adsorption of plasma proteins to the outer surface of the partition wall. Also, to promote the formation of dense connective tissue in the hollow space, the inner surface of the partition wall may require not only the properties suitable for the adsorption of plasma proteins, but also the properties suitable for the formation of dense connective tissue by fibroblasts. Furthermore, to promote the formation of dense connective tissue in the hollow space, the surface of the partition wall may also require the properties that promote the supply of fibroblasts themselves, that is, the properties suitable for the accumulation of somatic stem cells that can be induced into fibroblasts.

For example, when the contact angle with water of the surface of the tissue body formation device is outside the above-mentioned range that is suitable for adsorption of plasma proteins, plasma proteins are less likely to be adsorbed to the surface of the partition wall. This may promote the material exchange on the outer surface of the partition wall. The promoted material exchange may limit stratification of plasma proteins on the outer surface of the partition wall and the inner surfaces of the through-holes, and promote the supply of plasma proteins into the hollow space. Also, even when the contact angle with water of the surface that is in contact with the hollow space is outside the above-mentioned range that is suitable for adsorption of plasma proteins, the formation of dense connective tissue may be promoted as long as surface free energy suitable for the formation of dense connective tissue is provided. Furthermore, a surface that is suitable for fibroblasts to form dense connective tissue is a reaction surface that is suitable for causing this surface to be recognized by fibroblasts as the surface of foreign matter. As such, this surface differs from a surface suitable for plasma protein adsorption.

From the above viewpoint, the inventor of the present application, while intensively researching the relationship between the proportion of dense connective tissue in a connective tissue body and the properties of the surface of the partition wall, found that the proportion of dense connective tissue in a connective tissue body varies with the properties of the surface of the partition wall. While classifying the properties of the partition wall surface based on the proportion of dense connective tissue, the inventor of the present application found that when a surface on which a connective tissue body grows satisfies the following three conditions, the proportion of dense connective tissue on this surface sharply increases.
[Condition 1] The compressive strength is 10 MPa or greater and 60 MPa or less.
[Condition 2] The contact angle with water is 90° or greater.
[Condition 3] The opening size of the through-holes is 0.02 mm or greater and 2.5 mm or less.

According to the tissue body formation device described above, since the opening size of the through-holes is 0.02 mm or greater and 2.5 mm or less, the living tissue material in the biological environment is supplied to the hollow space. Since the compressive strength of the non-degradable resin is 10 MPa or greater and 60 MPa or less, and the contact angle with water of the non-degradable resin is 90° or greater, the proportion of dense connective tissue in the dense section of the connective tissue body is improved.

A connective tissue body is tissue including collagen as the main component. The formation of a connective tissue body in the technique of the present disclosure may be the formation of a connective tissue body in an in-vivo environment, or may be the formation of a connective tissue body in an in-vitro environment. The living tissue material is a material required for the formation of tissue derived from a living body. The living tissue materials include animal cells; proteins; sugars, such as hyaluronic acid; cell growth factors, which promote the growth and differentiation of cells; and various physiologically active substances present in living bodies, such as cytokines. Animal cells include somatic stem cells, fibroblast cells, smooth muscle cells, ES cells, and iPS cells. Proteins include collagen and elastin. Examples of living tissue materials include materials derived from humans or non-human animals, such as mammals including dogs, cows, pigs, goats, and sheep, birds, fish, and others, and equivalent artificial materials. Examples of an environment in which a living tissue material is present include a human body and a living body of non-human animals, such as mammals including dogs, cows, pigs, goats, and sheep, birds, fish, and others; in particular, subcutaneous locations in extremity, shoulder, back, and abdomen, and abdominal cavity. Another example of an environment in which a living tissue material is present is an artificial environment containing a living tissue material.

With the tissue body formation device described above, a section of the connective tissue body that includes loose connective tissue is a loose section, and the surface of the partition wall that is in contact with the loose section has a contact angle with water that is less than a contact angle with water of the surface of the partition wall that is in contact with the dense section.

When the connective tissue body is used as a heart valve, the high density of the section of the connective tissue body that is used as the outer surface of the valve limits the penetration of the blood into the connective tissue body. As such, the connective tissue body is expected to limit thrombus formation. That is, when the connective tissue body is a heart valve, the valve is a dense section, and the surface of the partition wall that is in contact with the pericardium preferably satisfies [Condition 1] to [Condition 3]. When the connective tissue body is used as a blood vessel, the higher density of the section of the connective tissue body that is used as the inner lumen surface of the blood vessel is expected to limit thrombus formation. On the other hand, the lower density of the section of the connective tissue body that is used as the outer circumference surface of the blood vessel facilitates the infiltration by surrounding tissue in the living body. This is expected to promote engraftment. That is, when the connective tissue body is a blood vessel, the inner lumen surface is a dense section, and the surface of the partition wall that is in contact with the inner lumen surface preferably satisfies [Condition 1] to [Condition 3]. In contrast, the outer circumference surface of the blood vessel is a loose section, and the surface of the partition wall that is in contact with the outer circumference surface of the blood vessel preferably has a smaller contact angle with water. As such, whether dense connective tissue is included in a predetermined section of the connective tissue body or whether loose connective tissue is included in another predetermined section can vary depending on the application of the connective tissue body.

According to the tissue body formation device described above, the surface of the partition wall that is to be in contact with a loose section of the connective tissue body has a smaller contact angle with water than the surface of the partition wall that is to be in contact with a dense section of the connective tissue body. This configuration allows the dense connective tissue to be unevenly distributed in predetermined sections of the connective tissue body and also allows the loose connective tissue to be unevenly distributed in predetermined sections of the connective tissue body.

In the above tissue body formation device, the exterior portion may include a first metal plate including a first inner surface and the through-holes and a second metal plate including a second inner surface and the through-holes. The first metal plate is joined to the second metal plate in a separable manner such that the first inner surface and the second inner surface face each other. The first inner surface is a curved recess surface that is recessed away from the second inner surface in the first metal plate, and has a shape that winds in a spiral in the first metal plate. The second inner surface is a curved recess surface that is recessed away from the first inner surface in the second metal plate, and has a shape that winds in a spiral in the second metal plate. The first inner surface and the second inner surface separate the single hollow space that winds in a spiral from the environment. The through-holes may be located in and around the first inner surface in the first metal plate and also located in and around the second inner surface in the second metal plate.

According to the above configuration, each of the first and second inner surfaces has a spiral shape. This allows the hollow space defined by the first and second inner surfaces to be extended in the extending direction of the hollow space within the tissue body formation device that has a limited, predetermined size. Also, the first metal plate is joined to the second metal plate in a separable manner such that the first inner surface and the second inner surface face each other. As a result, the separation of the first metal plate and the second metal plate facilitates the removal of the connective tissue body. Furthermore, the through-holes are located not only in the first inner surface but also in a section around the first inner surface of the first metal plate. This reduces the possibility that through-holes are not formed in the edges of the first inner surface due to any processing errors of the through-holes. As for the second inner surface, the possibility that through-holes are not formed in the edges of the second inner surface due to any processing errors of through-holes is reduced in the same manner. That is, this allows the hollow space to be uniform as viewed from the environment in which the living tissue material is present. This configuration is particularly suitable for a configuration in which the presence or absence of the through-holes can depend on the processing precision, that is, a tissue body formation device that includes small through-holes at fine intervals.

In the above tissue body formation device, the partition wall may have a spiral shape that conforms to the hollow space, and include an interior portion that defines the hollow space in a space surrounded by the first inner surface and the second inner surface. The surface in contact with the dense section may include a surface of the interior portion.

According to the above tissue body formation device, the hollow space is defined to have the shape of a tube sandwiched between the exterior portion and the interior portion, so that a tubular connective tissue body is formed. The dense connective tissue is formed at the inner circumference surface of the tubular connective tissue body. Accordingly, when the connective tissue body is used as a blood vessel, the section that serves as the inner lumen surface of the blood vessel has a high density.

In the above tissue body formation device, the length between opening edges of mutually adjacent through-holes may be 0.2 mm or greater, a ratio of an opening area of all of the through-holes that are disposed in a unit area of an outer surface of the partition wall to the unit area of the outer surface of the partition wall may be an opening occupancy rate, a ratio of a length of the opening edges of all of the through-holes that are disposed in a unit area of an outer surface of the exterior portion to the unit area of the outer surface of the exterior portion may be a partition surface density, the opening occupancy rate may be 4% or greater and 70% or less, and the partition surface density may be 0.8/mm or greater.

Since the length between mutually adjacent through-holes is 0.2 mm or greater, in the section of the partition wall located between one opening and another opening, the formation of collagen fibers tends to start from separate starting points at the edge of one opening and the edge of the other opening. In the section of the hollow space facing an opening, the larger the opening, the slower the formation of the connective tissue body. When the opening occupancy rate is 70% or less and the partition surface density is 0.8/mm or greater, the formation of a connective tissue body is unlikely to be delayed in the section facing the opening. The above configurations limit the lengthening of the period in which the tissue body formation device is implanted in a biological environment.

Additionally, while classifying the partition surface density based on the degree to which the proportion of dense connective tissue increases, the inventor of the present application has found that the degree to which the proportion of dense connective tissue increases also varies with the partition surface density. The inventor of the present application has found that when the tissue body formation device satisfies the following three conditions, the proportion of dense connective tissue is further effectively increased.
[Condition 4] The length between mutually adjacent through-holes is 0.2 mm or greater.
[Condition 5] The opening occupancy rate is 4% or greater and 70% or less.
[Condition 6] The partition surface density is 0.8/mm or greater.

Thus, with the above configuration, the length between mutually adjacent through-holes is 0.2 mm or greater, the opening occupancy rate is 4% or greater and 70% or less, and the partition surface density is 0.8/mm or greater. As a result, the proportion of dense connective tissue in the connective tissue body is significantly increased.

In the above tissue body formation device, a shape of openings of the through-holes may include a shape of two or more line segments intersecting at one point.

For example, a T-shaped opening or an X-shaped opening is formed by two line segments intersecting at one point. A Y-shaped opening is formed by three line segments intersecting at one point. When two line segments intersecting at one point form one line segment pair, E-shaped, F-shaped, H-shaped, and K-shaped openings each have two or more line segment pairs.

As for an opening having the shape of two or more line segments intersecting at one point, a greater number of line segments intersecting at one point increases the length of the opening edges in the unit area. In this manner, the above configuration has a greater partition surface density within the limitation of the length between mutually adjacent through-holes. This further limits the lengthening of the period in which the tissue body formation device is implanted in a biological environment.

In the above tissue body formation device, the shape of openings of the through-holes may represent visually perceivable information.

Examples of visual information include the manufacturer of the connective tissue body, the distributor of the connective tissue body, the site to which the connective tissue body is transplanted, the orientation of the connective tissue body when it is placed in the transplant site, and the section of the connective tissue body that is connected to the connection target of the transplant site. The outer surface of the connective tissue body formed by the tissue body formation device reflects the shape of the through-holes defined by the partition wall. The above configuration provides the outer surface of the connective tissue body with visual information without the need for additionally processing the connective tissue body. This achieves the indication of the origin of the connective tissue body and its quality assurance, and also provides the user with guidance regarding appropriate handling of the connective tissue body, without the need for additionally processing the connective tissue body.

A tissue body formation method for solving the above problems is a tissue body formation method for forming a connective tissue body in a tissue body formation device by implanting the tissue body formation device in an environment that is other than a human body and in which a living tissue material is present, and the tissue body formation device is the tissue body formation device described above.

### BRIEF DESCRIPTION OF DRAWINGS

Fig. 1 is a perspective view illustrating the structure of a tissue body formation device.
Fig. 2 is a plan view of the inner surface of an upper exterior portion as viewed from the inner side of the hollow space.
Fig. 3 is a plan view of the inner surface of a lower exterior portion as viewed from the inner side of the hollow space.
Fig. 4 is a plan view illustrating the structure of an interior member of the tissue body formation device.
Fig. 5 is a plan view illustrating the structure of openings of the tissue body formation device.
Fig. 6 is a process diagram illustrating the inside of the tissue body formation device in a tissue body forming process.
Fig. 7 is a process diagram illustrating the inside of the tissue body formation device in a tissue body forming process.
Fig. 8 is a process diagram illustrating a tissue body forming process of a tissue body formation device of a reference example.
Fig. 9 is a process diagram illustrating the inside of a tissue body formation device in a tissue body forming process.
Fig. 10 is a process diagram illustrating the inside of a tissue body formation device in a tissue body forming process.
Fig. 11 is a process diagram illustrating the inside of a tissue body formation device in a tissue body forming process.
Fig. 12 is a process diagram illustrating the inside of a tissue body formation device in a tissue body forming process.
Fig. 13 is a process diagram illustrating the inside of a tissue body formation device in a tissue body forming process.
Fig. 14 is a graph illustrating the relationship between a contact angle and a layer thickness ratio.
Fig. 15 is a graph illustrating the relationship between a partition surface density and a layer thickness ratio difference.
Fig. 16 is a plan view illustrating an example of a tissue body formation device in which the arrangement of through-holes is changed.
Fig. 17 is a plan view illustrating an example of a tissue body formation device in which the size of the through-holes is changed.
Fig. 18 is a plan view illustrating an example of a tissue body formation device in which the layer structure of the exterior portion is changed.
Fig. 19 is a plan view illustrating an example of a tissue body formation device in which the shape of the through-holes is changed.
Fig. 20 is a plan view illustrating an example of a tissue body formation device in which the configuration between through-holes is changed.
Fig. 21 is a plan view illustrating an example of a tissue body formation device in which the configuration between through-holes is changed.
Fig. 22 is a plan view illustrating an example of a tissue body formation device with an increased partition surface density.
Fig. 23 is a plan view illustrating an example of a tissue body formation device with an increased partition surface density.
Fig. 24 is a perspective view illustrating an example of a tissue body formation device with a modified device structure.
Fig. 25 is a perspective view illustrating an example of a tissue body formation device with a modified device structure.
Fig. 26 is a perspective view illustrating an example of a tissue body formation device with a modified device structure.
Fig. 27 is a perspective view illustrating an example of a tissue body formation device with a modified device structure.
Fig. 28 is a perspective view illustrating an example of a tissue body formation device with a modified device structure.
Fig. 29 is a perspective view illustrating an example of a tissue body formation device with a modified device structure.
Fig. 30 is a perspective view illustrating an example of a tissue body formation device with a modified device structure.
Fig. 31 is a perspective view illustrating an example of a tissue body formation device with a modified device structure.

### DESCRIPTION OF EMBODIMENTS

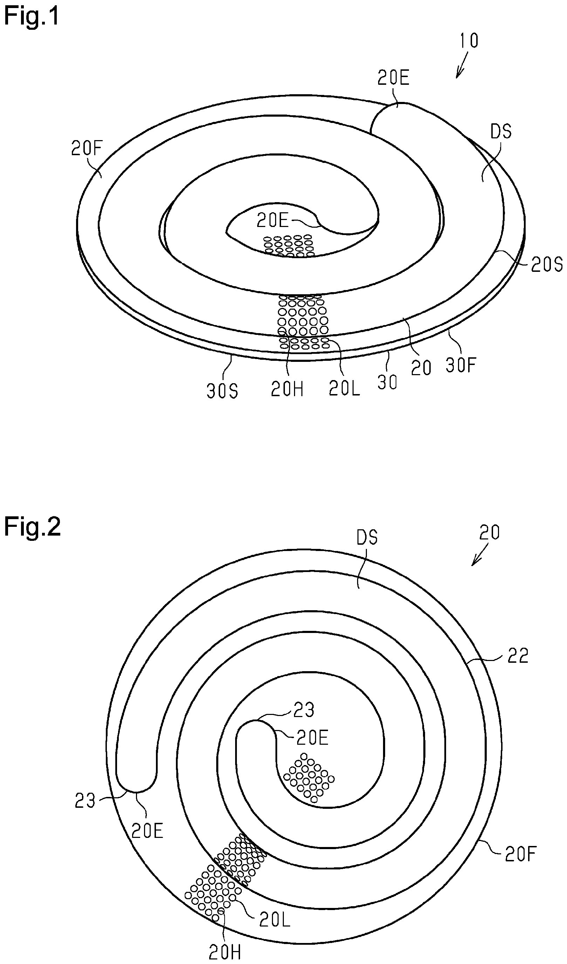

Referring to Figs. 1 to 15, a tissue body formation device according to one embodiment is now described.

### [Tissue Body Formation Device]

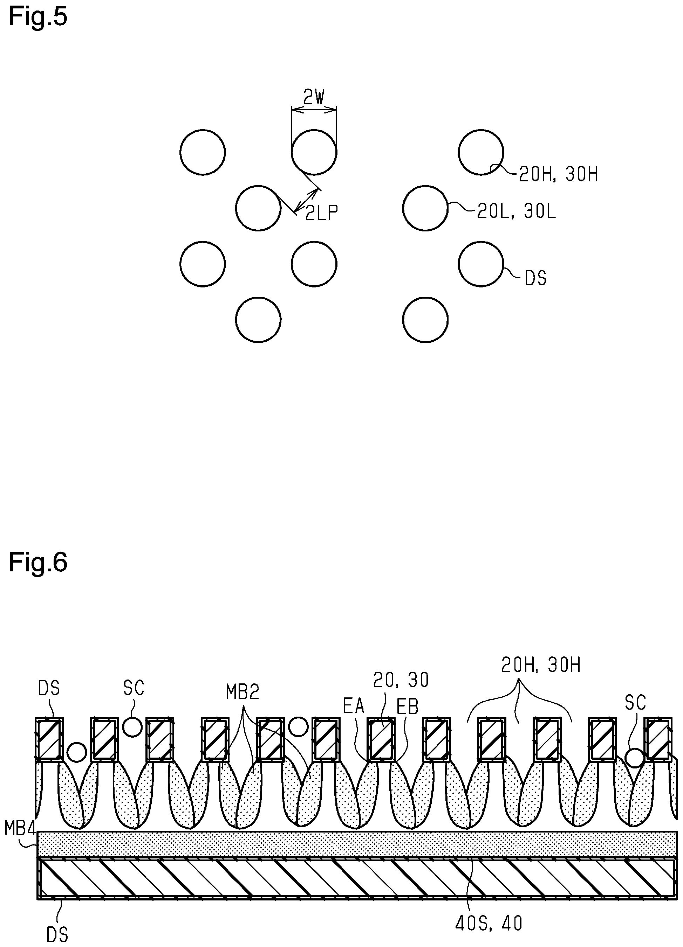

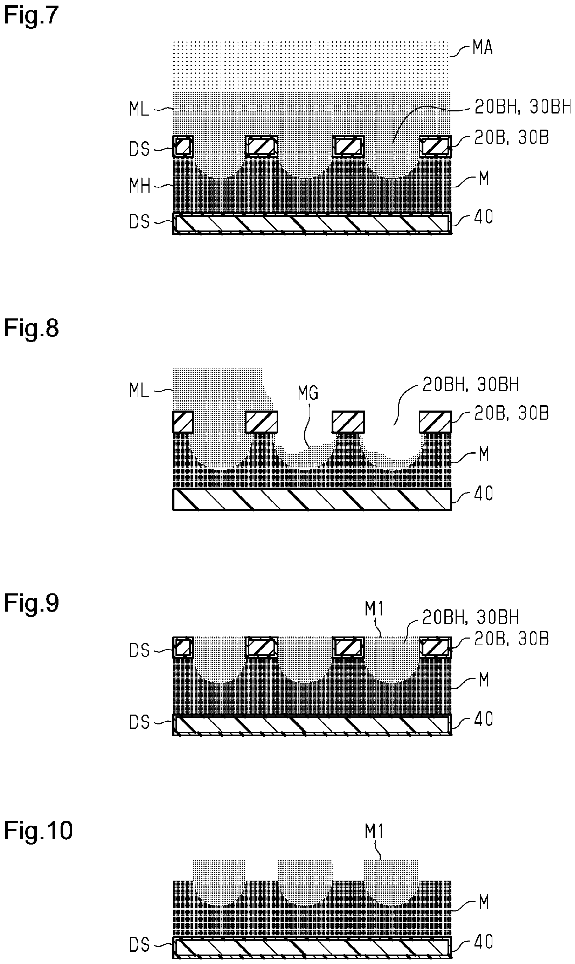

Fig. 1 illustrates a tissue body formation device, which is to be implanted in an environment in which a living tissue material is present to form a connective tissue body M (see Fig. 7). As described above, living tissue materials include animal cells; proteins; sugars, such as hyaluronic acid; cell growth factors, which promote the growth and differentiation of cells; and various physiologically active substances present in living bodies, such as cytokines. Animal cells include somatic stem cells SC (see Fig. 6), fibroblast cells, smooth muscle cells, ES cells, and iPS cells. Proteins include collagen and elastin. Examples of living tissue materials include materials derived from humans or non-human animals, such as mammals including dogs, cows, pigs, goats, and sheep, birds, fish, and others, and equivalent artificial materials. Examples of an environment in which a living tissue material is present include a human body and a living body of non-human animals, such as mammals including dogs, cows, pigs, goats, and sheep, birds, fish, and others; in particular, subcutaneous locations in extremity, shoulder, back, and abdomen, and abdominal cavity. Another example of an environment in which a living tissue material is present is an artificial environment containing a living tissue material. The connective tissue bodies M include irregular connective tissue, such as dermis, blood vessels, and perichondrium; and regular connective tissue, such as tendons, ligaments, corneas, and fascia.

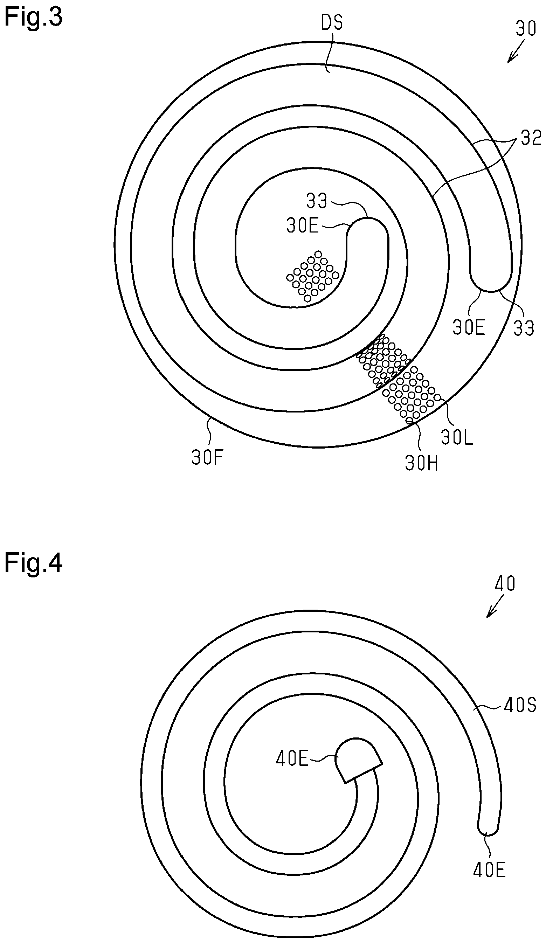

The tissue body formation device includes an exterior portion 10. The exterior portion 10 forms a part of a partition wall. The tissue body formation device separates an environment in which a living tissue material is present from a hollow space, which is ring-shaped and has two or more turns. The hollow space is a space for forming a connective tissue body M. The exterior portion 10 is the outer shell of the partition wall that defines the hollow space. The exterior portion 10 defines a space accommodating an interior portion 40 (see Fig. 4). The interior portion 40 is ring-shaped and has two or more turns in conformance with the ring shape of the hollow space. The interior portion 40 is an inner shell within the partition wall, which defines the hollow space.

The exterior portion 10 includes an upper exterior portion 20 and a lower exterior portion 30. The upper exterior portion 20 is joined to the lower exterior portion 30 so as to be separable from the lower exterior portion 30. The upper exterior portion 20 and the lower exterior portion 30 have shapes that are symmetrical with respect to a horizontal plane that is the joining surface of the upper exterior portion 20 and the lower exterior portion 30. The upper exterior portion 20 includes an upper ring-shaped portion 22 with two or more turns (see Fig. 2) for defining the hollow space. The lower exterior portion 30 also includes a lower ring-shaped portion 32 with two or more turns (see Fig. 3) for defining the hollow space.

The upper exterior portion 20 includes an upper ring-shaped flange 20F at the outermost circumference of the upper exterior portion 20. The upper ring-shaped flange 20F extends over the entire circumference of the upper exterior portion 20. The lower exterior portion 30 also includes a lower ring-shaped flange 30F at the outermost circumference of the lower exterior portion 30. The lower ring-shaped flange 30F extends over the entire circumference of the lower exterior portion 30. The upper ring-shaped flange 20F and the lower ring-shaped flange 30F facilitate implantation of the tissue body formation device, placement of the tissue body formation device, and removal of the tissue body formation device.

### [Non-degradable Resin Coating DS]

The upper exterior portion 20 includes an upper metal plate 20B (see Fig. 7), which is an example of a first metal plate, and a non-degradable resin coating DS, which covers the metal surface of the upper metal plate 20B.

The upper metal plate 20B is made of a material that is compatible with living tissues. An example of the material of the upper metal plate 20B is selected from the group consisting of stainless steel, titanium, titanium-nickel alloy, and cobalt-chromium alloy. In one example, the upper metal plate 20B has a thickness of 0.2 mm or greater and 2 mm or less.

The non-degradable resin coating DS is made of a material having a lower compressive strength than the upper metal plate 20B. Conversely, the upper metal plate 20B has a higher compressive strength than the non-degradable resin coating DS. The upper metal plate 20B has a higher rigidity than the non-degradable resin coating DS. The surface of the non-degradable resin coating DS has a greater contact angle with water than the metal surface of the upper metal plate 20B.

In the upper exterior portion 20, the upper metal plate 20B has a function of limiting deformation of the fine shape of the upper exterior portion 20. The non-degradable resin coating DS functions to (i) impart softness to the surface of the upper exterior portion 20 and (ii) increase the contact angle of the surface of the upper exterior portion 20 with water. In the connective tissue body M, a section that includes more dense connective tissue MH (see Fig. 7) than loose connective tissue ML (see Fig. 7) is a dense section that includes dense connective tissue MH. In the connective tissue body M, a section that includes more loose connective tissue ML than dense connective tissue MH is a loose section that includes loose connective tissue ML. The above-mentioned functions of (i) imparting softness and (ii) increasing the contact angle with water facilitate the formation of dense sections in the sections of the connective tissue body M in contact with the exterior portion 10.

Dense connective tissue MH is connective tissue that contains sufficiently more type I collagen than type III collagen. Loose connective tissue ML is connective tissue that contains type III collagen and type I collagen in equal degrees, or contains more type III collagen than type I collagen. Type I collagen is mainly made of dense collagen fibers with their longitudinal directions aligned. Type III collagen is mainly made of loose collagen fibers with their longitudinal directions randomly arranged.

In one example, the non-degradable resin coating DS has a thickness of 10 µm or greater and 100 µm or less. The thickness of the non-degradable resin coating DS is substantially uniform over the entire upper exterior portion 20. The difference between the maximum thickness and the minimum thickness of the non-degradable resin coating DS may be within 10% of the average value of the thickness of the non-degradable resin coating DS, for example.

An example of the non-degradable resin forming the non-degradable resin coating DS may be selected from the group consisting of polymethylpentene resin, polyfluoroethylene resin, polypropylene resin, polyethylene resin, silicon resin, and a mixed resin of these and other resin components. Examples of the mixed resin include a mixture of polymethylpentene resin and polypropylene resin, and a mixture of polyfluoroethylene resin and polyethylene resin. When 100 parts by mass of non-degradable resin forms the non-degradable resin coating DS, the non-degradable resin forming the non-degradable resin coating DS contains less than 50 parts by mass of the other resin components forming the mixed resin.

The polymethylpentene resin includes a constitutional unit derived from 4-methylpentene-1 in its resin components. The polymethylpentene resin includes polymethylpentene and a methylpentene copolymer. The methylpentene copolymer contains a constitutional unit derived from 4-methylpentene-1 and other constitutional units. Other constitutional units include constitutional units derived from olefins that are other than 4-methylpentene-1 and have 2 to 10 carbon atoms. Examples of methylpentene copolymers include ethylene/methylpentene copolymer, 1-butene/methylpentene copolymer, and propylene/methylpentene copolymer.

The polyfluoroethylene resin includes a constitutional unit derived from fluoroethylene in its resin components. An example of polyfluoroethylene resin is selected from the group consisting of polytetrafluoroethylene, polyvinylidene fluoride, polychlorotrifluoroethylene, ethylene/tetrafluoroethylene copolymer, and a mixed resin thereof.

In addition to polyethylene, the polyethylene resin includes a copolymer of a constitutional unit derived from ethylene and other constitutional units. Other constitutional units in the polyethylene resin include constitutional units derived from olefins having 3 to 10 carbon atoms. In addition to polypropylene, the polypropylene resin includes a copolymer of a constitutional unit derived from propylene and other constitutional units. Other constitutional units in the polypropylene resin include constitutional units derived from olefins that are other than propylene and have 2 to 10 carbon atoms.

The non-degradable resin forming the non-degradable resin coating DS satisfies [Condition 1] and [Condition 2] below.
[Condition 1] The compressive strength is 10 MPa or greater and 60 MPa or less.
[Condition 2] The contact angle of the surface with water is 90° or greater.

The compressive strength of [Condition 1] is a value measured in accordance with ASTM D695 (Standard Test Method for Compressive Properties of Rigid Plastics).

The contact angle with water of [Condition 2] is a static contact angle determined by a droplet method using water. The contact angle with water is the angle between the surface of the non-degradable resin coating DS and the droplet of water that has landed on the non-degradable resin coating DS. The contact angle with water is determined using the tangent at an end point of the water droplet assuming that the vicinity of the end point of the droplet is a part of a circle.

The lower exterior portion 30 includes a lower metal plate 30B, which is an example of a second metal plate, and a non-degradable resin coating DS, which covers the surface of the lower metal plate 30B. As with the upper exterior portion 20, the non-degradable resin coating DS of the lower exterior portion 30 covers the lower metal plate 30B with a substantially uniform thickness over the entire lower exterior portion 30.

The material forming the lower metal plate 30B in the lower exterior portion 30 is selected from the range described as examples of the material forming the upper metal plate 20B. The non-degradable resin forming the non-degradable resin coating DS in the upper exterior portion 20 is also selected from the range described as examples of the non-degradable resin forming the non-degradable resin coating DS in the upper exterior portion 20. As with the non-degradable resin coating DS in the upper exterior portion 20, the non-degradable resin forming the non-degradable resin coating DS in the lower exterior portion 30 satisfies the above-mentioned [Condition 1] and [Condition 2].

The material forming the upper exterior portion 20 and the material forming the lower exterior portion 30 may be the same material or different materials. The use of the same material increases the affinity between the upper and lower exterior portions 20 and 30, thereby improving the adhesion between the upper and lower exterior portions 20 and 30 in the tissue body formation device. The use of mutually different materials allows the upper side and the lower side of the exterior portion 10 to have different functions based on different materials, allowing the tissue body formation device to be multifunctional.

The non-degradable resin coating DS that serves as the outer surface of the upper exterior portion 20 is to be in contact with the biological environment MA (see Fig. 7), which is an environment in which a living tissue material is present. The non-degradable resin coating DS that serves as the inner surface of the upper exterior portion 20 is to be in contact with the living tissue material entering the hollow space. The non-degradable resin coating DS that serves as the outer surface of the lower exterior portion 30 is to be in contact with the environment in which a living tissue material is present. The non-degradable resin coating DS that serves as the inner surface of the upper exterior portion 20 is to be in contact with the living tissue material entering the hollow space.

As illustrated in Fig. 2, the inner surface of the upper exterior portion 20 is circular as viewed from inside the hollow space. The inner surface of the upper exterior portion 20 has an upper ring-shaped curved recess surface covered with a non-degradable resin coating DS. The upper ring-shaped curved recess surface, which is an example of the first inner surface, is the inner surface of the upper ring-shaped portion 22 that is recessed in a direction away from the lower exterior portion 30.

The upper ring-shaped curved recess surface is shaped to wind around the center of the inner surface of the upper exterior portion 20. The upper ring-shaped curved recess surface is a semi-cylindrical surface that winds around the winding center, and each successive turn is progressively farther from the center. In other words, the upper ring-shaped curved recess surface has a spiral shape. Fig. 2 illustrates an example of the upper ring-shaped curved recess surface being a semi-cylindrical surface having the shape of a spiral with two turns.

In one example, the upper ring-shaped curved recess surface may be formed by pressing the inner surface of the upper metal plate 20B, which forms the upper exterior portion 20, outward. The upper ring-shaped curved recess surface includes two end portions 20E in the extending direction of the upper ring-shaped curved recess surface. The two end portions 20E define the ends 23 of the hollow space in the extending direction of the hollow space. One of the two end portions 20E supports one end of the interior portion 40. The interior portion 40 supported by the end portion 20E has an interior surface 40S (see Fig. 4) separated from the upper ring-shaped curved recess surface. The gap between the interior surface 40S of the interior portion 40 and the upper ring-shaped curved recess surface forms the upper half of the hollow space defined by the exterior portion 10. The double-turn spiral shape of the upper ring-shaped curved recess surface increases the length of the cylindrical hollow space in the extending direction of the hollow space within the disc-shaped exterior portion 10.

The upper exterior portion 20 includes multiple upper through-holes 20H, which are examples of through-holes. The upper through-holes 20H extend between the outside of the upper exterior portion 20 and the hollow space. The non-degradable resin coating DS forming the upper exterior portion 20 forms the inner surface of the upper exterior portion 20, the outer surface of the upper exterior portion 20, and the inner surfaces defining the upper through-holes 20H. Each upper through-hole 20H includes an upper outer opening, which opens at the upper outer surface 20S, and an upper inner opening, which opens to the hollow space. The upper through-hole 20H may have a circular hole shape, for example. The outline line defining each upper outer opening in the upper outer surface 20S is referred to as an upper partition line 20L, which is an example of an opening edge. When the upper through-holes 20H have a circular hole shape, the upper partition line 20L has a circular shape. Figs. 1 and 2 illustrate only some of the upper through-holes 20H for convenience of explaining the structure of the tissue body formation device.

The upper through-holes 20H are formed over the entire upper exterior portion 20. That is, the upper through-holes 20H are formed in the upper ring-shaped portion 22 and also around the upper ring-shaped portion 22. To form the upper through-holes 20H, various types of hole processing, such as etching the upper metal plate 20B or laser drilling the upper metal plate 20B, and the application of the non-degradable resin coating DS to the upper metal plate 20B after hole processing may be performed. Alternatively, the upper through-holes 20H may be formed by first performing various types of hole processing, such as etching the upper metal plate 20B or laser drilling the upper metal plate 20B. Then, the application of the non-degradable resin coating DS to the upper metal plate 20B after hole processing and further hole processing of the non-degradable resin coating DS may be performed.

A smaller size of the upper through-holes 20H and finer intervals between the upper through-holes 20H increase the possibility of processing errors in the dimensions and arrangement of the upper through-holes 20H in the edges of the processing area in which the upper through-holes 20H are provided. As compared to a configuration that includes upper through-holes 20H only in the upper ring-shaped portion 22, a configuration that includes upper through-holes 20H over the entire upper exterior portion 20 reduces the possibility that sections without upper through-holes 20H are unevenly present in the edges of the upper ring-shaped portion 22. Reducing the possibility that sections without upper through-holes 20H are unevenly present is particularly advantageous with a configuration including small upper through-holes 20H at fine intervals.

As illustrated in Fig. 3, the inner surface of the lower exterior portion 30 has a circular shape as viewed from inside the hollow space. The inner surface of the lower exterior portion 30 has a lower ring-shaped curved recess surface covered with a non-degradable resin coating DS. The lower ring-shaped curved recess surface, which is an example of the second inner surface, is the inner surface of the lower ring-shaped portion 32 that is recessed in a direction away from the upper exterior portion 20. The lower ring-shaped curved recess surface is shaped to wind around the center of the inner surface of the lower exterior portion 30. The shape of the lower ring-shaped curved recess surface is symmetrical to the upper ring-shaped curved recess surface with respect to a horizontal plane. That is, the lower ring-shaped curved recess surface is a semi-cylindrical surface that winds around the winding center, and each successive turn is progressively farther from the center. In other words, the lower ring-shaped curved recess surface has a spiral shape. Fig. 3 illustrates an example of the lower ring-shaped curved recess surface being a semi-cylindrical surface having the shape of a spiral with two turns.

In the same manner as the upper ring-shaped curved recess surface, the lower ring-shaped curved recess surface may be formed by pressing the inner surface of the lower exterior portion 30, for example. The lower ring-shaped curved recess surface includes two end portions 30E in the extending direction of the upper ring-shaped curved recess surface. The two end portions 30E define the ends 33 of the hollow space in the extending direction of the hollow space. One of the two end portions 30E supports one end of the interior portion 40. The interior portion 40 supported by the end portion 30E has the interior surface 40S (see Fig. 4) separated from the lower ring-shaped curved recess surface. The gap between the interior surface 40S of the interior portion 40 and the lower ring-shaped curved recess surface forms the lower half of the hollow space defined by the exterior portion 10. Like the upper ring-shaped curved recess surface, the double-turn spiral shape of the lower ring-shaped curved recess surface increases the length of the cylindrical hollow space in the extending direction of the hollow space within the disc-shaped exterior portion 10.

The lower exterior portion 30 includes multiple lower through-holes 30H, which are examples of through-holes. The lower through-holes 30H extend between the outside of the lower exterior portion 30 and the hollow space. The non-degradable resin coating DS forming the lower exterior portion 30 forms the inner surface of the lower exterior portion 30, the outer surface of the lower exterior portion 30, and the inner surfaces defining the lower through-holes 30H. Each lower through-hole 30H includes a lower outer opening, which opens at the lower outer surface 30S, and a lower inner opening, which opens to the hollow space. Like the upper through-holes 20H, the lower through-holes 30H may have a circular hole shape, for example. The outline line defining each lower outer opening in the lower outer surface 30S is referred to as a lower partition line 30L, which is an example of an opening edge. When the lower through-holes 30H have a circular hole shape, the lower partition line 30L has a circular shape. Fig. 3 illustrates only some of the lower through-holes 30H for convenience of explaining the structure of the tissue body formation device.

The lower through-holes 30H are formed over the entire lower exterior portion 30. That is, the lower through-holes 30H are formed in the lower ring-shaped portion 32 and also around the lower ring-shaped portion 32. As compared to a configuration that includes lower through-holes 30H only in the lower ring-shaped portion 32, a configuration that includes lower through-holes 30H over the entire lower exterior portion 30 reduces the possibility that sections without lower through-holes 30H are unevenly present in the edges of the lower ring-shaped portion 32. Reducing the possibility that sections without lower through-holes 30H are unevenly present is particularly advantageous with a configuration including small lower through-holes 30H at fine intervals.

As described above, the upper ring-shaped curved recess surface of the upper exterior portion 20 and the lower ring-shaped curved recess surface of the lower exterior portion 30 form one inner ring-shaped cylindrical surface surrounding the interior surface 40S (see Fig. 4), which is the surface of the interior portion 40. One inner ring-shaped cylindrical surface formed by the upper and lower ring-shaped curved recess surfaces has a spiral shape and extends continuously forming gaps in the radial direction of the exterior portion 10. The inner ring-shaped cylindrical surface is formed by the upper ring-shaped portion 22 and the lower ring-shaped portion 32. The outer surface of the upper ring-shaped portion 22 and the outer surface of the lower ring-shaped portion 32 form an outer ring-shaped cylindrical surface, which conforms to the inner ring-shaped cylindrical surface.

A line segment corresponding to one complete loop of the upper ring-shaped portion 22 is spaced apart from another line segment corresponding to one complete loop of the upper ring-shaped portion 22 by a gap of 0.5 mm or greater, for example, in the radial direction of the exterior portion 10. Likewise, a line segment corresponding to one complete loop of the lower ring-shaped portion 32 is spaced apart from another line segment corresponding to one complete loop of the lower ring-shaped portion 32 by a gap of 0.5 mm or greater, for example, in the radial direction of the exterior portion 10.

A configuration including upper through-holes 20H in the entire upper exterior portion 20 and lower through-holes 30H in the entire lower exterior portion 30 includes through-holes 20H and 30H extending vertically through a section between a line segment corresponding to one complete loop in the ring-shaped portion and another line segment corresponding to one complete loop in the ring-shaped portion. The configuration that includes through-holes 20H and 30H not only in the line segments of the ring-shaped portions but also in the section between the line segments allows the substantially entire outer ring-shaped cylindrical surface, including the sections facing the section between the line segments of the ring-shaped portion, to be perceived as a uniform cylindrical surface as viewed from the environment in which a living tissue material is present. Furthermore, the configuration that includes through-holes 20H and 30H also in the section between the line segments of the ring-shaped portions allows the substantially entire inner ring-shaped cylindrical surface, including the sections facing the section between the line segments of the ring-shaped portion, to be perceived as a uniform cylindrical surface as viewed from the environment in which a living tissue material is present.

Thus, in terms of the processing, such as the fine placement of small through-holes 20H and 30H, the configuration having through-holes 20H and 30H in the entire exterior portion 10 reduces the possibility of absence of through-holes 20H and 30H in the edges of the upper ring-shaped portion 22 and the edges of the lower ring-shaped portion 32. Also, in terms of the tissue formation, such as the entry of the living tissue material into the tissue body formation device, the configuration having through-holes 20H and 30H in the entire exterior portion 10 reduces non-uniformity of the connective tissue body M.

### [Hollow Space]

As illustrated in Fig. 4, the interior portion 40 is a ring-shaped columnar member having two or more turns. The interior portion 40 is shaped to wind around the center of the exterior portion 10. The interior portion 40 is a columnar member that winds around the winding center, and each successive turn is progressively farther from the center. In other words, the interior portion has a spiral shape. The interior portion 40 includes two end portions 40E in the extending direction of the interior portion 40. One of the two end portions 40E is an enlarged portion having the largest outer diameter in the interior portion 40. The enlarged portion of the interior portion 40 is supported by the end portion 20E of the upper ring-shaped curved recess surface and the end portion 30E of the lower ring-shaped curved recess surface. The fit between the enlarged portion and the end portion 20E determines the distance between the interior surface 40S of the interior portion 40 and the inner surface of the upper exterior portion 20. The fit between the enlarged portion and the end portion 30E determines the distance between the interior surface 40S of the interior portion 40 and the inner surface of the lower exterior portion 30.

The gap between the inner surface of the upper exterior portion 20 and the interior surface 40S and the gap between the inner surface of the lower exterior portion 30 and the interior surface 40S form the cylindrical hollow space for forming a connective tissue body M. The thickness of the hollow space is also the thickness of the connective tissue body M filling the hollow space. The thickness of the hollow space corresponds to the thickness required for the connective tissue body M. The thickness of the hollow space may be 0.5 mm or greater and 5 mm or less, for example. A hollow space having a thickness of 0.5 mm or greater facilitates the formation of dense connective tissue MH over the entire connective tissue body M. A hollow space having a thickness of 5 mm or less reduces the likelihood of a section remaining in the hollow space without being filled with the connective tissue body M after the tissue body formation device has been implanted in the biological environment MA for about four weeks.

The inner surface of the upper exterior portion 20 is an uneven surface in which steps having the same depth as the upper through-holes 20H are formed in a smooth surface. The inner surface of the lower exterior portion 30 is an uneven surface in which steps having the same depth as lower through-holes 30H are formed in a smooth surface. In contrast, the interior surface 40S of the interior portion 40 is a sufficiently smooth surface having smaller steps than the inner surface of the upper exterior portion 20 and the inner surfaces of the lower through-holes 30H. The smooth interior surface 40S facilitates the removal of the interior portion 40 from the connective tissue body M.

The interior portion 40 includes an interior base and a non-degradable resin coating DS covering the surface of the interior base. As with the metal plates 20B and 30b, the interior base is made of a metal material that is compatible with living tissue. An example of the material of the interior base is selected from the group consisting of stainless steel, titanium, titanium-nickel alloy, and cobalt-chromium alloy.

As with the non-degradable resin coating DS of the exterior portion 10, the non-degradable resin coating DS of the interior portion 40 functions to (i) impart softness to the surface of the interior portion 40, and (ii) increase the contact angle of the surface of the interior portion 40 with water. The functions of (i) imparting softness and (ii) increasing the contact angle with water facilitate the formation of dense sections in the sections of the connective tissue body M in contact with the interior portion 40. As with the non-degradable resin coating DS of the exterior portion 10, the non-degradable resin forming the non-degradable resin coating DS of the interior portion 40 satisfies [Condition 1] and [Condition 2]. In one example, the non-degradable resin of the interior portion 40 is selected from the group consisting of polymethylpentene resin, polyfluoroethylene resin, polypropylene resin, polyethylene resin, silicon resin, and a mixed resin of these and other resin components.

In one example, the non-degradable resin coating DS of the interior portion 40 has a thickness of 10 µm or greater and 100 µm or less. The non-degradable resin coating DS of the interior portion 40 is made of a material having a lower compressive strength than the interior base. The non-degradable resin forming the non-degradable resin coating DS of the interior portion 40 may be the same as the non-degradable resin coating DS of the upper exterior portion 20, or may be different from the non-degradable resin coating DS of the upper exterior portion 20. Furthermore, the interior base of the interior portion 40 may be made of a non-degradable resin, or the interior base and the non-degradable resin coating DS may be made of the same non-degradable resin and form a single-layer structure.

The thickness of the upper exterior portion 20 and the thickness of the lower exterior portion 30 are the thickness of the exterior portion 10. The thickness of the upper exterior portion 20 is the depth of the upper through-holes 20H. The thickness of the lower exterior portion 30 is the depth of the lower through-holes 30H. When the living tissue material requires to be smoothly supplied toward the hollow space through the upper outer openings, the upper through-holes 20H preferably have a smaller depth and preferably have a depth of 2.0 mm or less, provided that the mechanical strength of the exterior portion 10 is maintained. When the living tissue material requires to be smoothly supplied toward the hollow space through the lower outer openings, the lower through-holes 30H preferably have a smaller depth and preferably have a depth of 2.0 mm or less, provided that the mechanical strength of the exterior portion 10 is maintained. When the thickness of the dense connective tissue MH in the connective tissue body M requires to be uniform, the dimensions such as the depth and opening diameter of the upper through-holes 20H are preferably equal to the dimensions of the lower through-holes 30H.

### [Connective Tissue Body Formation Method]

A method for forming a connective tissue body M using the tissue body formation device described above is now described. Examples of an environment in which a living tissue material is present include a human body and a living body of non-human animals, such as mammals including dogs, cows, pigs, goats, and sheep, birds, fish, and others; in particular, subcutaneous locations in extremity, shoulder, back, and abdomen, and abdominal cavity. Another example of an environment in which a living tissue material is present is an artificial environment containing a living tissue material. To implant the tissue body formation device in a living body, a minimal incision is first made into the living body under sufficient anesthesia. After the tissue body formation device is implanted, the wound is sutured.

First, in the tissue body formation device implanted in the living body, plasma proteins are adsorbed to the non-degradable resin coating DS that forms the outer surface of the exterior portion 10, and fibroblasts are adsorbed to the plasma proteins. Then, the living tissue material enters the hollow space through the upper through-holes 20H and the lower through-holes 30H. At this time, plasma proteins are adsorbed to the non-degradable resin coating DS that forms the inner surfaces of the upper through-holes 20H, the inner surfaces of the lower through-holes 30H, the inner surface of the upper exterior portion 20, and the inner surface of the lower exterior portion 30, and fibroblasts are adsorbed to the plasma proteins. The living tissue material entering the hollow space forms a connective tissue body M in the hollow space.

The hollow space for forming a connective tissue body M is surrounded by the non-degradable resin coating DS. Rather than the absorption of plasma proteins to the outer surface of the exterior portion 10, the formation of the connective tissue body M in the hollow space requires the subsequent adsorption of plasma proteins to the inner surface of the upper exterior portion 20 and the absorption of plasma proteins to the inner surface of the lower exterior portion 30.

The surface properties of the non-degradable resin coating DS that satisfies [Condition 2] differ from those that are ideal for facilitating the adsorption of plasma proteins. Rather, this non-degradable resin coating DS facilitates the material exchange at the outer surface of the exterior portion 10, the material exchange at the inner surfaces of the upper through-holes 20H, and the material exchange at the inner surfaces of the lower through-holes 30H. Such material exchange delays the adsorption of plasma proteins to the outer surface of the exterior portion 10, the inner surfaces of the upper through-holes 20H, and the inner surfaces of the lower through-holes 30H, and also limits the stratification of plasma proteins. As a result, the material exchange is considered to promote the supply of plasma proteins toward the hollow space through the upper through-holes 20H and the lower through-holes 30H.

After the adsorption of fibroblasts, which is expected to occur after the adsorption of plasma proteins, the non-degradable resin coating DS that satisfies [Condition 2] allows the fibroblasts to (ii) have a contact angle with water that is suitable for the growth of dense connective tissue MH. Thus, the non-degradable resin coating DS that satisfies [Condition 2] is expected to stimulate the fibroblasts to produce collagen and also provide the collagen with surface free energy that promotes the growth of dense connective tissue MH.

The upper and lower through-holes 20H and 30H form the passages that extend between the outside of the tissue body formation device and the hollow space. The opening edges EA and EB of the upper through-holes 20H (see Fig. 6) and the opening edges EA and EB of the lower through-holes 30H are corners that materials can easily reach from a wide area in the biological environment MA, as compared to simple flat surfaces. These corners are more likely to be recognized as foreign matter by fibroblasts than other parts of the tissue body formation device.

Since the non-degradable resin coating DS that satisfies [Condition 2] facilitates the material exchange, provides surface free energy suitable for the growth of dense connective tissue MH, and enhances the recognition of the corners as foreign matter as described above, it can shorten the formation time of the connective tissue body M in the hollow space. Moreover, thickening of dense connective tissue MH can also be achieved. Furthermore, the thickening of the dense connective tissue MH involves the fibroblasts being promoted to produce collagen. As such, in the process of forming a connective tissue body M, the degree of accumulation of somatic stem cells SC, which can be induced into fibroblasts, also increases.

The non-degradable resin coating DS that satisfies [Condition 1] provides (i) a soft surface for the living tissue material, as compared to a hard non-degradable resin coating DS with a compressive strength of 50 MPa or higher. A hard surface that does not satisfy [Condition 1] may allow the collagen fibers reaching the hard surface to be easily fixed regardless of the longitudinal directions of the collagen fibers. In contrast, the soft surface of the non-degradable resin coating DS may cause the longitudinal directions of the collagen fibers to be aligned with the collagen fibers such that the collagen fibers are stabilized on the surface of the non-degradable resin coating DS. That is, the soft surface of the non-degradable resin coating DS is expected to promote the growth of dense connective tissue MH. This may facilitate the stabilization of the structure having the soft surface as a foothold, thereby promoting the stratification of collagen fibers that form dense connective tissue MH. Additionally, the soft surface of the non-degradable resin coating DS allows the dense connective tissue MH to grow while being pressed against the non-degradable resin coating DS during the formation process of the connective tissue body M. This may further promote the growth of the dense connective tissue MH. Accordingly, the non-degradable resin coating DS that satisfies [Condition 1] further thickens the dense connective tissue MH in the connective tissue body M.

The tissue body formation device implanted in the biological environment MA is removed from the environment after a predetermined implant period, which is a period in which the connective tissue body M is formed, has elapsed. To remove the tissue body formation device from the biological environment MA, a minimal incision is first made into the living body under sufficient anesthesia. After the tissue body formation device is removed, the wound is sutured.

The connective tissue body M formed using the tissue body formation device has a cylindrical shape that conforms to the shape of the hollow space. The inner circumference surface of the connective tissue body M has a shape that conforms to the interior surface 40S. The inner diameter of the connective tissue body M corresponds to the outer diameter of the interior portion 40. The shape of the outer surface of the connective tissue body M conforms to the inner surface of the upper exterior portion 20 and the inner surface of the lower exterior portion 30. The outer diameter of the connective tissue body M corresponds to the inner diameter of the upper exterior portion 20 and the inner diameter of the lower exterior portion 30. The outer surface of the connective tissue body M has a minute uneven shape that conforms to the upper through-holes 20H and the lower through-holes 30H.

When the connective tissue body M is used for xenotransplantation, the connective tissue body M is preferably subjected to immunogen removal treatment, such as decellularization treatment, dehydration treatment, and fixation treatment, to limit rejection after transplantation. Examples of the decellularization treatment include ultrasonic treatment, surfactant treatment, and a treatment in which the extracellular matrix is eluted by treatment with enzyme, such as collagenase, and cleaned. The dehydration treatment is a treatment of washing the connective tissue body M with a water-soluble organic solvent, such as methanol, ethanol, and isopropyl alcohol. The fixation treatment is a treatment in which the connective tissue body M is immersed in an aldehyde compound, such as glutaraldehyde or formaldehyde.

### [Partition Surface Density and Contact Angle]

Various dimensional conditions that the exterior portion 10 satisfies are described below. Fig. 5 is a schematic diagram of the outer surface of the upper ring-shaped portion 22 and the outer surface of the lower ring-shaped portion 32 developed in a plane.

As illustrated in Fig. 5, the openings of the upper through-holes 20H and the openings of the lower through-holes 30H are circular regions. At least some of the numerous upper through-holes 20H and at least some of the numerous lower through-holes 30H are arranged such that the centers of the openings substantially coincide with lattice points of a square lattice. The diameter of each opening is an opening size 2W. The minimum dimension of mutually adjacent opening edges EA and EB is an inter-opening size 2LP, which is the distance between the opening edges. All of the upper through-holes 20H may be arranged such that the centers of the openings substantially coincide with lattice points of the square lattice. Likewise, all of the lower through-holes 30H may be arranged such that the centers of the openings substantially coincide with lattice points of the square lattice.

The area of a unit region of the outer surface of the exterior portion 10 is the unit area of the exterior portion 10. The total area of the openings occupying the unit region is the opening area defined by the openings. The ratio of the opening area to the unit area of the exterior portion 10 is the opening occupancy rate (%). The total length of the partition lines 20L, 30L within the unit region is the surface density of openings. The value obtained by dividing the surface density of the openings by the unit area of the exterior portion 10 is a partition surface density (/mm).

The unit region of the outer surface of the exterior portion 10 may be a unit repeating in the outer surface, for example. An example of a unit region of the outer surface of the exterior portion 10 is a rectangular region surrounded by four lattice points of the square lattice described above. The unit region of the outer surface of the exterior portion 10 may be a region including one opening and its surrounding area, a region including a group of openings and its surrounding area, or a rectangular region set in advance. When the unit region is a predetermined region that repeats irrespective of openings, the above-mentioned opening occupancy rate and the partition surface density are the average value of the opening occupancy rates in all unit regions and the average value of the partition surface densities in all unit regions.

When the dense connective tissue MH requires to be thicker, the opening size 2W, the inter-opening size 2LP, the opening occupancy rate, and the partition surface density preferably satisfy the following [Condition 3] to [Condition 6].
[Condition 3] Opening size 2W: 0.02 mm or greater and 2.5 mm or less
[Condition 4] Inter-opening size 2LP: 0.2 mm or greater
[Condition 5] Opening occupancy rate: 4% or greater and 70% or less
[Condition 6] Partition surface density: 0.8/mm or greater

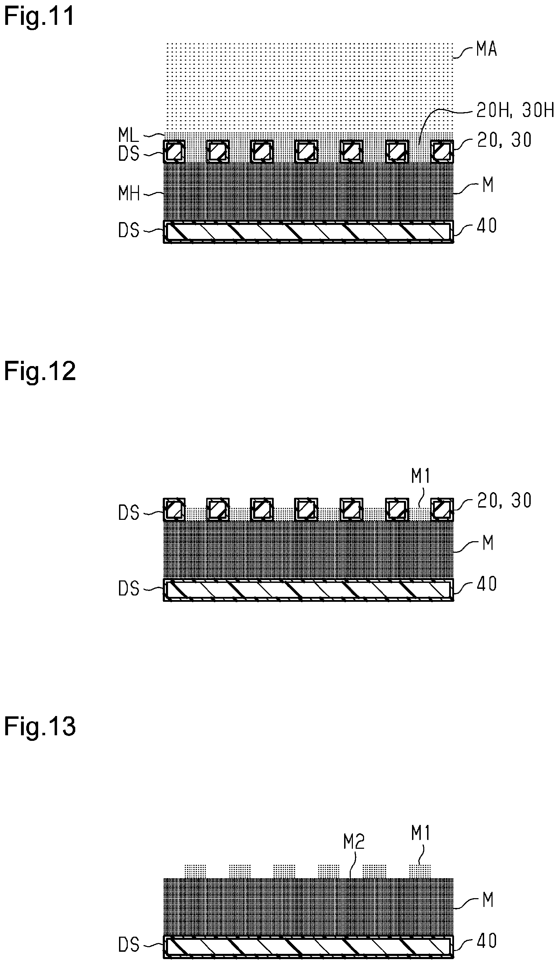

The minimum size of the opening through which the living tissue material can pass is about 0.01 mm. Thus, when the opening size 2W is 0.02 mm or greater, the living tissue material for forming the connective tissue body M sufficiently passes through the opening. An opening size 2W of 2.5 mm or less allows the protruding portions M1 (see Figs. 10 and 13), which conform to the shape of the upper through-holes 20H and the lower through-holes 30H, to be smaller in size in the connective tissue body M. This facilitates the removal of the tissue body formation device from the environment in which a living tissue material is present, and also allows the connective tissue body M to be easily peeled off from the tissue body formation device. Additionally, such a size can increase the surface flatness of the connective tissue body M and also reduce the possibility of the connective tissue body M developing a tendency that prevents the ring-shaped connective tissue body M from being changed into another shape, such as a linear shape.

An inter-opening size 2LP of 0.2 mm or greater means that the opening edge EA of one of mutually adjacent upper through-holes 20H is separate from the opening edge EB of the other. This also means that the opening edge EA of one of mutually adjacent lower through-holes 30H is separate from the opening edge EB of the other.

These opening edges EA and EB are corners that increase the adsorption probability for living tissue material as compared to the inner surface of the upper ring-shaped portion 22, the inner surface of the lower ring-shaped portion 32, the inner surfaces of the upper through-holes 20H, and the inner surfaces of the lower through-holes 30H. The opening edges EA and EB, which are corners, are more easily recognized as foreign matter than other sections of the tissue body formation device, and thus serve as separate starting points to promote the formation of the connective tissue body M. The opening edges EA and EB as corners separately start the formation of protruding tissue bodies MB2, which are connective tissue bodies M, from the opening edges EA and EB toward the inside of the hollow space. The two protruding tissue bodies MB2, which start to form from one structure located between openings, increase the formation speed of the connective tissue body M, as compared to a configuration in which one protruding tissue body MB2 starts to form from one structure. As compared to one protruding tissue body MB2, the two protruding tissue bodies MB2 increase the number of points at which collagen is produced to reach the non-degradable resin coating DS of the interior portion 40. This further promotes the growth of the dense connective tissue MH on the interior surface 40S of the interior portion 40. On the interior surface 40S of the interior portion 40, after an appropriate amount of plasma proteins supplied through the through-holes 20H and 30H is adsorbed, the non-degradable resin coating DS stimulates collagen production by the fibroblasts adsorbed to the plasma proteins. This advances the growth of a covering tissue body MB4, which is dense connective tissue MH. At this time, the short-term growth of the protruding tissue bodies MB2 and the covering tissue body MB4 promotes the accumulation of somatic stem cells SC in the hollow space.

In the process of coating the metal plates 20B and 30B with the non-degradable resin coating DS, the surface tension of the material forming the non-degradable resin coating DS may prevent, to some extent, the non-degradable resin coating DS from having a uniform thickness. For example, the surface tension of the material forming the non-degradable resin coating DS acts to promote aggregation of the material forming the non-degradable resin coating DS between two opening edges EA and EB. The surface tension of the material forming the non-degradable resin coating DS acts to shape the surface of the non-degradable resin coating DS into a hemispherical shape that connects two opening edges EA and EB to each other. The surface of the non-degradable resin coating DS that has a hemispherical shape may prevent, to some extent, the opening edges EA and EB from functioning as separate corners. A smaller inter-opening size 2LP increases the likelihood of the surface of the non-degradable resin coating DS having a hemispherical shape.

In this regard, with a configuration that satisfies [Condition 1] to [Condition 6], a combination of an inter-opening size 2LP of 0.2 mm or greater and the small surface tension of the non-degradable resin coating DS reduces the likelihood of the surface of the non-degradable resin coating DS having a hemispherical shape. That is, with a tissue body formation device that satisfies [Condition 1] and [Condition 2] and has an inter-opening size 2LP of 0.2 mm or greater, the non-degradable resin coating DS is more likely to have a uniform thickness that allows the opening edges EA and EB to function as separate corners. Also, the non-degradable resin coating DS causing the covering tissue body MB4 to grow on the interior surface 40S promotes the accumulation of somatic stem cells SC in the hollow space. This allows the inter-opening size 2LP to be smaller while allowing the opening edges EA and EB to function as separate corners.

The formation of the connective tissue body M with the tissue body formation device involves competition between the outer surface of the exterior portion 10 and the interior of the hollow space. Even if the opening occupancy rate is mutually equal, a configuration with a small number of large openings is likely to cause the outer surface of the exterior portion 10 to be covered with collagen film because of the excessively large inter-opening size 2LP. As a result, collagen film is likely to close the openings before the hollow space is filled with a connective tissue body M. Alternatively, plasma proteins, somatic stem cells SC, and fibroblasts are not easily supplied to the hollow space, and the entry of living tissue material into the hollow space is excessively delayed. In contrast, when a large number of openings are provided and the size of the openings is sufficiently small, the smaller inter-opening size 2LP reduces the likelihood of the outer surface of the exterior portion 10 being covered by collagen film. Also, this maintains a sufficient number of starting points for the formation of dense connective tissue MH. When the interior surface 40S of the interior portion 40 is made of a non-degradable resin that satisfies [Condition 1] and [Condition 2], the growth of dense connective tissue MH is promoted, the accumulation of somatic stem cells SC is accelerated, and the production of collagen by fibroblasts is also accelerated. As a result, the openings are unlikely to be closed before a connective tissue body M is formed, and thus the hollow space is more likely to be filled with a connective tissue body M. As such, [Condition 3] to [Condition 6] may be considered as inseparable conditions and represent a configuration including a large number of appropriately small openings. In particular, [Condition 6] that the partition surface density is 0.8/mm or greater is a condition that further increases the effect of thickening the dense connective tissue MH.

Referring to Figs. 7 to 10, the process of forming a connective tissue body M using a tissue body formation device that satisfies [Condition 1] to [Condition 5] and has a partition surface density of less than 0.8/mm is described. Fig. 8 illustrates a process of forming a connective tissue body M using a device that does not include a non-degradable resin coating DS, as a tissue body formation device of a reference example. Referring to Figs. 11 to 13, a process of forming a connective tissue body M using an example in which [Condition 1] to [Condition 5] are satisfied and the partition surface density is 0.8/mm or greater, that is, a tissue body formation device that satisfies [Condition 1] to [Condition 6] is described.

As illustrated in Fig. 7, the biological environment MA supplies the living tissue material to the hollow space even if the tissue body formation device does not satisfy [Condition 6]. The outer surface of the exterior portion 10, which is continuously in contact with the biological environment MA, promotes material exchange on the outer surface. As a result, the outer surface is covered with loose connective tissue ML whose main component is type III collagen produced from the living tissue material. Also, the inside of the upper through-holes 20H and a portion between the upper inner openings and the interior portion 40 are filled with loose connective tissue ML. The inside of the lower through-holes 30H and a portion between the lower inner openings and the interior portion 40 are also filled with loose connective tissue ML. The loose connective tissue ML is shaped to protrude toward the interior portion 40 from the upper inner openings of the upper through-holes 20H and protrude toward the interior portion 40 from the lower inner openings of the lower through-holes 30H.

As for the tissue body formation device that does not satisfy [Condition 6], the dense connective tissue MH including type I collagen as the main component is still formed between the upper exterior portion 20 and the interior portion 40 and between the lower exterior portion 30 and the interior portion 40. The dense connective tissue MH fills the space between the inner surface of the upper exterior portion 20 and the interior portion 40, and also fills the space between the inner surface of the lower exterior portion 30 and the interior portion 40.

In contrast, the space between the upper inner openings of the upper through-holes 20H and the interior portion 40 is filled with a laminate of the loose connective tissue ML and the dense connective tissue MH. The space between the upper inner openings of the lower through-holes 30H and the interior portion 40 is also filled with a laminate of the loose connective tissue ML and the dense connective tissue MH. The thickness of the dense connective tissue MH is smallest in the sections of the dense connective tissue MH that face the upper inner openings of the upper through-holes 20H and the sections that face the lower inner openings of the lower through-holes 30H.

As illustrated in Fig. 8, with the tissue body formation device that does not satisfy [Condition 6] and does not include a non-degradable resin coating DS, loose connective tissue ML spreads to a greater extent between the upper inner openings and the interior portion 40, as compared to a configuration including a non-degradable resin coating DS. In other words, when a tissue body formation device that does not satisfy [Condition 6] and does not include a non-degradable resin coating DS is used, the sections of the dense connective tissue MH that face the upper inner openings are very thin. Likewise, the sections of the dense connective tissue MH that face the lower inner openings are also very thin as compared to a configuration that includes a non-degradable resin coating DS.

When the dense connective tissue MH is thin and the loose connective tissue ML is thick in the sections facing the upper and lower inner openings, the loose connective tissue ML is more likely to split in the hollow space when the tissue body formation device is removed from the biological environment MA. In other words, when a tissue body formation device that does not satisfy [Condition 6] and does not include a non-degradable resin coating DS is used, a thin section MG is likely to be formed by the splitting of the loose connective tissue ML at the sections that face the upper inner openings and the outer inner openings.

In this regard, as illustrated in Fig. 9, as long as the configuration includes a non-degradable resin coating DS, the dense connective tissue MH is thick and the loose connective tissue ML is thin, even if the configuration does not satisfy [Condition 6]. This limits the splitting of the loose connective tissue ML in the connective tissue body M. As illustrated in Fig. 10, the connective tissue body M separated from the upper exterior portion 20 and the lower exterior portion 30 includes protruding portions M1, which have a shape that conforms to the inner surfaces of the upper through-holes 20H and the inner surfaces of the lower through-holes 30H and are made of loose connective tissue ML.

As illustrated in Fig. 11, the outer surface of the exterior portion 10 that satisfies [Condition 1] to [Condition 6] is covered with loose connective tissue ML, in the same manner as the exterior portion 10 that does not satisfy [Condition 6]. Furthermore, the inside of the upper through-holes 20H and the inside of the lower through-holes 30H are also filled with the loose connective tissue ML. In contrast, most of the space between the lower inner openings of the lower through-holes 30H and the interior portion 40 and the space between the upper inner openings of the upper through-holes 20H and the interior portion 40 is filled with dense connective tissue MH. That is, providing the non-degradable resin coating DS and having a partition surface density of 0.8/mm or greater thicken the dense connective tissue MH in the space between the lower inner openings of the lower through-holes 30H and the interior portion 40 and the space between the upper inner openings of the upper through-holes 20H and the interior portion 40.

As illustrated in Fig. 12, in the tissue body formation device that satisfies [Condition 1] to [Condition 6], the loose connective tissue ML is less likely to split in the sections facing the upper inner openings and the outer inner openings. As illustrated in Fig. 13, the connective tissue body M separated from the upper and lower exterior portions 20 and 30 includes protruding portions M1, which have a shape that conforms to the inner surfaces of the upper through-holes 20H and the inner surfaces of the lower through-holes 30H and are made of loose connective tissue ML. With the tissue body formation device that satisfies [Condition 1] to [Condition 6], the dense connective tissue MH forms a cylindrical surface M2 that conforms to the inner surface of the upper exterior portion 20 and the inner surface of the lower exterior portion 30 and has a substantially uniform outer diameter. This increases the toughness of the entire connective tissue body M.

### [Experimental Examples]

Experimental examples of the non-degradable resin coating DS of the tissue body formation device are now described.

As the upper and lower through-holes 20H and 30H of the tissue body formation devices of the experimental examples, circular holes were formed at lattice points of a square lattice over the entire surface of the exterior portion 10. As the experimental examples for the surface texture test, the compressive strength, contact angle with water, opening size 2W, inter-opening size 2LP, opening occupancy rate, and partition surface density were changed within the following formation conditions for the surface texture test. Multiple tissue body formation devices were thus prepared. As for the experimental examples for the surface texture test, the experimental examples had different compressive strengths and contact angles with water, and the same opening size 2W, inter-opening size 2LP, opening occupancy rate, and partition surface density. As the experimental examples for the hole size test, the compressive strength, contact angle with water, opening size 2W, inter-opening size 2LP, opening occupancy rate, and partition surface density were changed within the following formation conditions for the hole size test. Multiple tissue body formation devices were thus prepared. As for the experimental examples for the hole size test, the experimental examples had different opening sizes 2W, inter-opening sizes 2LP, opening occupancy rates, and partition surface densities, and the same compressive strength and contact angle with water.

### [Formation Conditions for Surface Texture Test]

· Metal plate: 1 mm thick stainless steel plate
· Compressive strength: 10 MPa or greater and 90 MPa or less
· Contact angle with water: 70° or greater and 145° or less
· Thickness of hollow space: 2.0 mm
· Opening size 2W: 0.02 mm or greater and 2.5 mm or less
· Inter-opening size 2LP: 0.1 mm or greater and 3.5 mm or less
· Opening occupancy rate: 4% or greater and 70% or less
· Partition surface density: 0.3/mm or greater and 20/mm or less
· Duration of implant: 4 weeks

### [Formation Conditions for Hole Size Test]

· Metal plate: 1 mm thick stainless steel plate
· Compressive strength, contact angle with water: [11 MPa, 110°] [55 MPa, 92°]
· Thickness of hollow space: 2.0 mm
· Opening size 2W: 0.4 mm or greater and 2.5 mm or less
· Inter-opening size 2LP: 0.2 mm or greater and 3.5 mm or less
· Opening occupancy rate: 10% or greater and 50% or less
· Partition surface density: 0.3/mm or greater and 4.0/mm or less
· Duration of implant: 4 weeks

For the tissue body formation devices of the experimental examples, non-degradable resin coatings DS were formed using mutually different non-degradable resins and surface treatments to obtain mutually different compression strengths and contact angles with water. The combination of compressive strength and contact angle with water is indicated as [Compressive strength, contact angle] and listed below together with the non-degradable resin used to obtain the tissue body formation device of the experimental example.

As a hydrophilic treatment to lower the contact angle with water, oxygen plasma was used to introduce hydroxyl groups to the surface of the non-degradable resin coating DS. The contact angle with water was adjusted by increasing the treatment time of the hydrophilic treatment to lower the contact angle with water. As a water repellent treatment to increase the contact angle with water, fluorine plasma was used to introduce a perfluoro group to the surface of the non-degradable resin coating DS. The contact angle with water was adjusted by increasing the treatment time of the water repellent treatment to increase the contact angle with water. Also, as a water repellent treatment for increasing the contact angle with water to 120° or greater, the surface structure of the non-degradable resin coating DS other than the inner surfaces of the through-holes 20H and 30H was subjected to a treatment for forming an uneven structure of projections arranged at intervals of several hundred micrometers.

To measure the compressive strength, the non-degradable resins of the experimental examples were used to form test pieces for compressive strength measurement. A method according to ASTM D695 (Standard Test Method for Compressive Properties of Rigid Plastics) was used to obtain the compressive strength of the test pieces for compressive strength measurement as the compressive strength of the tissue body formation device of each experimental example. To measure the contact angle with water, a sample piece for contact angle measurement was obtained by coating a flat metal plate with the non-degradable resin of each experimental example. Then, in an environment of 25°C, 2 µL of water was dropped on the surface of the sample piece for contact angle measurement, and the contact angle measured using the tangent at the end point of the droplet was obtained as the contact angle with water of the tissue body formation device of the experimental example.
· [10 MPa, 120°]: water repellent silicon resin
· [10 MPa, 95°]: silicon resin
· [10 MPa, 80°]: hydrophilic silicon resin
· [10 MPa, 70°]: hydrophilic silicon resin
· [11 MPa, 135°]: water repellent polytetrafluoroethylene
· [11 MPa, 110°]: polytetrafluoroethylene
· [11 MPa, 90°]: hydrophilic polytetrafluoroethylene
· [11 MPa, 82°]: hydrophilic polytetrafluoroethylene
· [45 MPa, 105°]: water repellent polypropylene
· [45 MPa, 95°]: polypropylene
· [45 MPa, 75°]: hydrophilic polypropylene
· [55 MPa, 135°]: water repellent polymethylpentene
· [55 MPa, 115°]: water repellent polymethylpentene
· [55 MPa, 92°]: polymethylpentene
· [55 MPa, 80°]: hydrophilic polymethylpentene
· [90 MPa, 138°]: water repellent polymethyl methacrylate
· [90 MPa, 95°]: water repellent polymethyl methacrylate
· [90 MPa, 70°]: polymethyl methacrylate

To form the connective tissue body M, a dog's body was used as the environment in which a living tissue material is present. The tissue body formation devices of experimental examples of mutually different formation conditions were implanted in the biological environment MA, and connective tissue bodies M were thus obtained from the tissue body formation devices of experimental examples.

For the evaluation of the tissue body formation devices, the layer thickness ratio RL (RL = H1/H0), which is the ratio of the layer thickness H1 of the dense connective tissue MH to the distance H0 between the upper inner opening and the outer surface of the interior portion 40, was determined for the connective tissue bodies M obtained using the tissue body formation devices of the experimental examples. The distance H0 between the upper inner opening of the upper through-hole 20H and the outer surface of the interior portion 40 corresponds to the thickness of the connective tissue body M.

To measure the layer thickness ratio RL, the connective tissue body M obtained from the tissue body formation device of each experimental example was fixed in 4% PFA at a temperature of 4°C for 24 hours. Then, sections were prepared from the paraffin-embedded block of the connective tissue body M such that the thickness of the sections was 3 µm or greater and 5 µm or less in the longitudinal direction of the connective tissue body M. Then, the sections were subjected to HE staining, MT staining, and SR staining, and the stained sections were observed under a microscope, and the sections after SR staining were observed under a polarizing microscope. It was observed that the sections obtained from the tissue body formation device of the experimental example were stained pink by HE staining, blue by MT staining, and red by SR staining. That is, the connective tissue body M obtained from the tissue body formation device of the experimental example was observed to contain collagen fibers.

In the polarizing microscope observation after SR staining, sections of the connective tissue body M formed between the upper inner openings of the upper through-holes 20H and the outer surface of the interior portion 40 were used, and the layer thickness H1 of the dense connective tissue MH was measured from the outer surface of the interior portion 40 to the upper inner openings of the upper through-holes 20H. At this time, the sections were observed at intervals of 100 µm from the outer surface of the interior portion 40 toward the upper inner openings of the upper through-holes 20H, and a region in which type I collagen, which exhibits a bright yellow to orange color, was more abundant than type III collagen, which exhibits a green color, was identified as dense connective tissue MH. A region in which type III collagen, which exhibits a green color, was equal to or greater than type I collagen in amount, which exhibits a yellow to orange color, was identified as loose connective tissue ML.

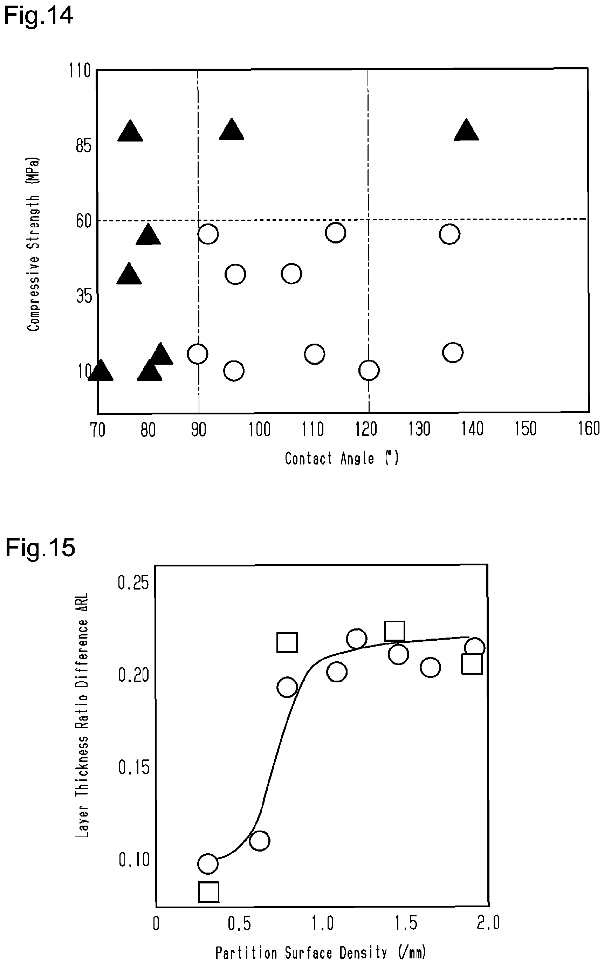

Fig. 14 illustrates the relationship between the layer thickness ratio RL, contact angle with water, and compressive strength of the connective tissue bodies M obtained from the tissue body formation devices of the experimental examples. Fig. 15 illustrates the relationship between the layer thickness ratio difference ΔRL, which is the degree of increase of the layer thickness ratio RL, and the partition surface density. Fig. 14 illustrates the results of the experimental examples with an opening size 2W of 0.4 mm, an inter-opening size 2LP of 0.2 mm, an opening occupancy rate of 40%, and a partition surface density of 4/mm as formation conditions for surface texture test.

In Fig. 14, among the experimental examples for surface texture, the experimental examples having a layer thickness ratio RL of 0.6 or greater are indicated by open circles, and the experimental examples having a layer thickness ratio RL of 0.2 or greater and 0.4 or less are indicated by black triangles. In Fig. 15, among the text examples for hole size, the experimental examples with which the compressive strength and contact angle with water are [11 MPa, 115°] are indicated by white squares, and experimental examples with [55 MPa, 92°] are indicated by open circles. The layer thickness ratio difference ΔRL is the difference value between the smallest layer thickness ratio RL and the largest layer thickness ratio RL among the experimental examples for surface texture test that satisfy [Condition 1] and [Condition 2].

As illustrated in Fig. 14, the layer thickness ratio RL of the connective tissue body M sharply increases from a low level of 0.4 or less to a high level of 0.6 or greater when the contact angle with water becomes 90° or greater and the compressive strength becomes 60 MPa or less. That is, the layer thickness ratio RL in the connective tissue body M sharply increases when the contact angle with water increases and the compressive strength decreases so that [Condition 1] that the compressive strength is 10 MPa or greater and 60 MPa or less and [Condition 2] that the contact angle with water is 90° or greater are satisfied. The ranges that satisfy [Condition 1] and [Condition 2] have higher layer thickness ratios RL of the connective tissue body M than the ranges that do not satisfy [Condition 1] and [Condition 2]. Such a sharp increase in the layer thickness ratio RL and the maintenance of a high layer thickness ratio RL suggest the presence of a surface texture that is suitable for the formation of the dense connective tissue MH and that the suitable surface texture satisfies [Condition 1] and [Condition 2]. Furthermore, a configuration in which the surface that defines the hollow spaces has a surface texture that satisfies [Condition 1] and [Condition 2] has a higher proportion of dense connective tissue MH than a configuration that defines the hollow space with a surface having a surface texture that does not satisfy [Condition 1] and [Condition 2]. The examples illustrated in Fig. 14 are typical examples identified from the formation conditions for a surface texture test. With other dimensional conditions among the formation conditions for surface texture test, it was found that the boundary at which the layer thickness ratio RL sharply increases is the same as the typical example, although the absolute values of the layer thickness ratio RL are different.

Fig. 15 illustrates that the layer thickness ratio difference ΔRL of the connective tissue bodies M, that is, the degree of increase in the layer thickness ratio RL caused by satisfying [Condition 1] and [Condition 2] sharply increases as the partition surface density increases. Specifically, the layer thickness ratio difference ΔRL of the connective tissue bodies M sharply increases when [Conditions 1] to [Conditions 6] described above are satisfied. The increased layer thickness ratio difference ΔRL remains high as long as the partition surface density is 0.8/mm or greater. Such a sharp increase in the layer thickness ratio difference ΔRL suggests the contribution of the two opening edges EA and EB serving as separate production points, not only in terms of the formation time of the connective tissue body M, but also in terms of the degree of thickening of the dense connective tissue MH in an environment that satisfies [Condition 1] and [Condition 2].

With the experimental example for which the implant duration was shortened to two weeks, the outer surface of the connective tissue body M was found to include pockets, which were depressions surrounded by the openings of the through-holes 20H and 30H and the dense connective tissue MH. Using the connective tissue body M having the pockets, sections were prepared in the same manner as in the evaluation of the dense connective tissue MH. Then, using the sections of connective tissue body M, HE staining, MT staining, SR staining, mesenchymal stem cell staining using CD90 and CD105 as mesenchymal stem cell markers, and multifunctional stem cell staining using SSEA4 and SSEA3 as pluripotent stem cell markers were performed.

As a result of HE staining, MT staining, and SR staining, dense connective tissue MH surrounding the pockets of the connective tissue body M was observed. The connective tissue body M also had voids that were sufficiently smaller than the pockets, and dense connective tissue MH surrounding the voids was also observed. Additionally, loose connective tissue ML was observed filling the pockets of the connective tissue body M and also filling the voids scattered within the connective tissue M. As a result of staining of mesenchymal stem cells, mesenchymal stem cell marker-positive cells were observed in the pockets of the connective tissue body M and also in the voids scattered within the connective tissue body M. Also, as a result of staining of the multifunctional stem cells, the cells positive for the pluripotent stem cell marker were observed in the pockets of the connective tissue body M and also in the voids scattered within the connective tissue body M. It was also observed that the experimental examples having a larger proportion of the layer thickness H1 of the dense connective tissue MH had a greater number of positive cells.

The embodiments described above have the following advantageous effects.
(1) Since the opening size 2W of the upper through-holes 20H is 0.02 mm or greater and 2.5 mm or less, and the opening size 2W of the lower through-holes 30H is 0.02 mm or greater and 2.5 mm or less, the living tissue material in the biological environment MA is supplied to the hollow space. Since the compressive strength of the non-degradable resin is 10 MPa or greater and 60 MPa or less, and the contact angle with water of the non-degradable resin coating DS is 90° or greater, the proportion of dense connective tissue MH in the connective tissue body M is improved.
(2) In the surface of the tissue body formation device, the surface that is to be in contact with a loose section of the connective tissue body M may have a smaller contact angle with water than the surface of the partition wall that is to be in contact with a dense section of the connective tissue body M. This configuration allows the dense connective tissue MH to be unevenly distributed in predetermined sections of the connective tissue body M and also allows the loose connective tissue ML to be unevenly distributed in predetermined sections of the connective tissue body M.
(3) The section of the upper metal plate 20B around the upper ring-shaped curved recess surface includes upper through-holes 20H in the same manner as the upper ring-shaped curved recess surface. The section of the lower metal plate 30B around the lower ring-shaped curved recess surface includes lower through-holes 30H in the same manner as the lower ring-shaped curved recess surface. This reduces the possibility that through-holes 20H, 30H are not formed in the edges of the ring-shaped curved recess surface due to any processing errors of the through-holes 20H, 30H. That is, this allows the hollow space to be uniform as viewed from the environment in which a living tissue material is present. This configuration is particularly suitable for a structure in which the presence or absence of the through-holes 20H, 30H can depend on the processing precision, that is, a tissue body formation device that includes small through-holes 20H and 30H at fine intervals.
(4) The hollow space is defined to have the shape of a tube sandwiched between the exterior portion 10 and the interior portion 40, so that a tubular connective tissue body M is formed. The dense connective tissue MH is formed at the inner circumference surface of the tubular connective tissue body M. Accordingly, when the connective tissue body M is used as a blood vessel, the section that serves as the inner lumen surface of the blood vessel has a high density.
(5) A configuration that satisfies [Condition 3] to [Condition 6] significantly increases the proportion of dense connective tissue MH in the connective tissue body M.

The above embodiments may be modified and implemented as follows.

### [Arrangement of Through-holes 20H and 30H]

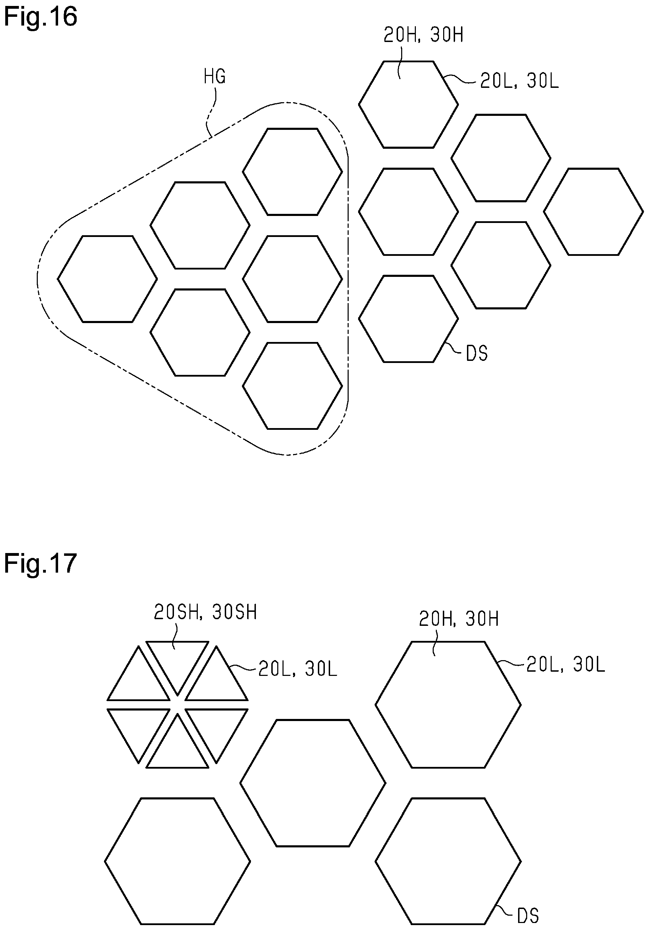

· As illustrated in Fig. 16, the upper through-holes 20H and the lower through-holes 30H formed in the exterior portion 10 may include groups each consisting of multiple through-holes 20H, 30H. For example, six through-holes 20H, 30H defining hexagonal openings are arranged at lattice points forming a hexagonal lattice, and these six through-holes 20H, 30H form one hole group HG. The exterior portion 10 may include multiple hole groups HG each consisting of six through-holes 20H, 30H. To increase the partition surface density, the through-holes 20H, 30H are preferably arranged in each hole group HG in a closest packed manner.

· In the above modification 1, the length between one hole group HG consisting of multiple through-holes 20H, 30H and another hole group HG consisting of multiple through-holes 20H, 30H may be longer or shorter than the length between the openings in one hole group HG. When the length between one hole group HG and another hole group HG is longer than the length in one hole group HG, the structure between one hole group HG and another hole group HG compensates for a decrease in the mechanical strength of the exterior portion 10 caused by a shorter length between the openings in one hole group HG.

· The arrangement of the openings defined by the upper through-holes 20H and the lower through-holes 30H may have regularity such that they are located at lattice points of a square lattice, lattice points of a rectangular lattice, lattice points of a hexagonal lattice, or may have irregularity. When the openings defined by the upper through-holes 20H and the lower through-holes 30H are arranged irregularly, a rectangular region having a side of 10 mm is defined as a unit region of the outer surface of the exterior portion 10.

### [Sizes of Through-Holes 20H and 30H]

· As illustrated in Fig. 17, the tissue body formation device may include two or more types of through-holes 20H and 30H having mutually different sizes. For example, the tissue body formation device may further include upper small through-holes 20SH and lower small through-holes 30SH in addition to the upper through-holes 20H and the lower through-holes 30H. Each upper small through-hole 20SH defines an opening smaller than the opening of an upper through-hole 20H in the upper exterior portion 20. Each lower small through-hole 30SH defines an opening smaller than the opening of a lower through-hole 30H in the lower exterior portion 30. In a similar manner as the above modification, the arrangement of the upper small through-holes 20SH and the lower small through-holes 30SH may include groups each consisting of multiple small through-holes 20SH, 30SH. The outline line of each hole group HG consisting of multiple small through-holes 20SH, 30SH may form an upper partition line 20L or a lower partition line 30L in the unit region. The two or more types of through-holes 20H, 30H, 20SH, and 30SH having mutually different sizes may be arranged in the exterior portion 10 so as to satisfy [Condition 3] to [Condition 6] as a group. This still has an advantageous effect equivalent to (5) above.

### [Layer Configuration of Exterior Portion 10]

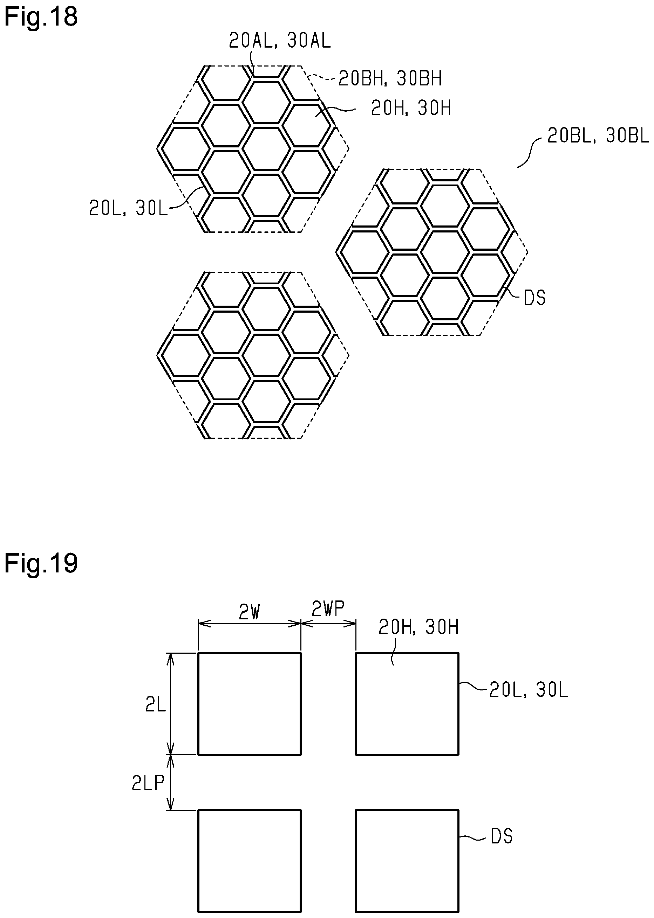

· As illustrated in Fig. 18, the upper exterior portion 20 may include two layers of an upper outer layer member 20BL, which includes upper through-holes 20H, and an upper inner layer member 20AL, which is fixed to the inner side of the upper outer layer member 20BL. The upper inner layer member 20AL includes upper through-holes 20H so as to satisfy the above Conditions 3 to 6. The upper outer layer member 20BL defines the openings of the upper outer layer through-holes 20BH, which are larger than the openings of the upper through-holes 20H. Of the openings defined by the upper through-holes 20H, the small openings located in the openings of the upper outer layer through-holes 20BH may be formed to satisfy [Condition 3] to [Condition 6] described above with respect to the outer surface of the exterior portion 10. The lower exterior portion 30 may also include two layers of a lower outer layer member 30BL, which includes lower outer layer through-holes 30BH, and a lower inner layer member 30AL, which includes lower through-holes 30H.

### [Shape of Through-Hole]

· As illustrated in Fig. 19, the upper through-holes 20H and the lower through-holes 30H formed in the exterior portion 10 may be rectangular and located at lattice points of a square lattice. The length of one side of the rectangular opening is the opening size 2W, and the length of another side perpendicular to the one side is the opening size 2L. In the extending direction of one side of the rectangular openings, the shortest distance between the openings is the inter-opening size 2LP. In the extending direction of another side of the rectangular opening, the shortest distance between the openings is the inter-opening size 2WP. When the opening sizes 2L and 2W satisfy [Condition 3], the inter-opening sizes 2LP and 2WP satisfy [Condition 4], and the configuration further satisfies [Condition 5] and [Condition 6], this configuration has an advantageous effect equivalent to (5) described above.

· The shape of the openings defined by the upper through-holes 20H and the shape of the openings defined by the lower through-holes 30H may be polygonal with rounded corners, elliptical, or other different geometric configurations. The shape of the openings defined by the upper through-holes 20H and the shape of the openings defined by the lower through-holes 30H may be an irregular shape different from a geometric shape, or may be a combination of two or more shapes selected from a group of multiple geometric shapes and multiple irregular shapes.

· The shape of the openings defined by the upper through-holes 20H and the shape of the openings defined by the lower through-holes 30H may represent visual information, such as letters, pictures, emojis, designs, and symbols. Visual information is information that can be identified by the user's sense of sight. For example, when the shape of the openings represents the manufacturer or distributor of the connective tissue body M as visual information, the shape of the protruding portions M1 of the connective tissue body M also reflects the visual information indicating the manufacturer or distributor. This achieves the indication of the origin of the connective tissue body M and its quality assurance without the need for additionally processing the connective tissue body M. For example, when the shape of the openings represents the transplant site as visual information, the shape of the protruding portions M1 of the connective tissue body M also reflects visual information indicating the transplant site. For example, when the shape of the openings represents the orientation such as the arrangement and connection of the connective tissue body M at the transplant site as visual information, the shape of the protruding portions M1 of the connective tissue body M also reflects the visual information indicating the orientation. This advantageously provides the user with guidance regarding the appropriate handling of the connective tissue body M, without the need for additionally processing the connective tissue body M.

### [Configuration between Through-Holes 20H and 30H]

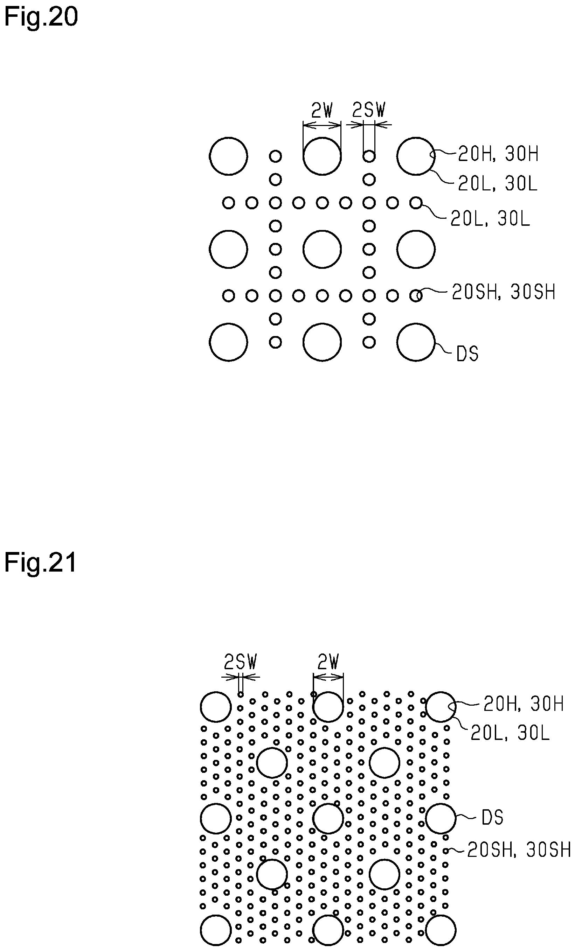

· As illustrated in Fig. 20, the exterior portion 10 may include upper small through-holes 20SH between mutually adjacent upper through-holes 20H. Each upper small through-hole 20SH has a smaller opening size 2SW than the upper through-holes 20H. The upper small through-holes 20SH may be located on the lattice lines of the square lattice having lattice points on which the upper through-holes 20H are located. The two types of through-holes 20H and 20SH located on the unit lattice may satisfy the above [condition 3] to [condition 6]. Similarly, the exterior portion 10 may include lower small through-holes 30SH between mutually adjacent lower through-holes 30H. Each lower small through-hole 30SH has a smaller opening size 2SW than the lower through-holes 30H. The lower small through-holes 30SH may be located on the lattice lines of the square lattice having lattice points on which the lower through-holes 30H are located. The two types of through-holes 30H and 30SH located on the unit lattice may satisfy the above [condition 3] to [condition 6].

· As illustrated in Fig. 21, the upper small through-holes 20SH may be scattered so as to fill the spaces between mutually adjacent upper through-holes 20H. The arrangement of the upper small through-holes 20SH may be regular or irregular. The lower small through-holes 30SH may also be scattered so as to fill the spaces between the mutually adjacent lower through-holes 30H. The arrangement of the lower small through-holes 30SH may be regular or irregular.

### [Partition Surface Density]

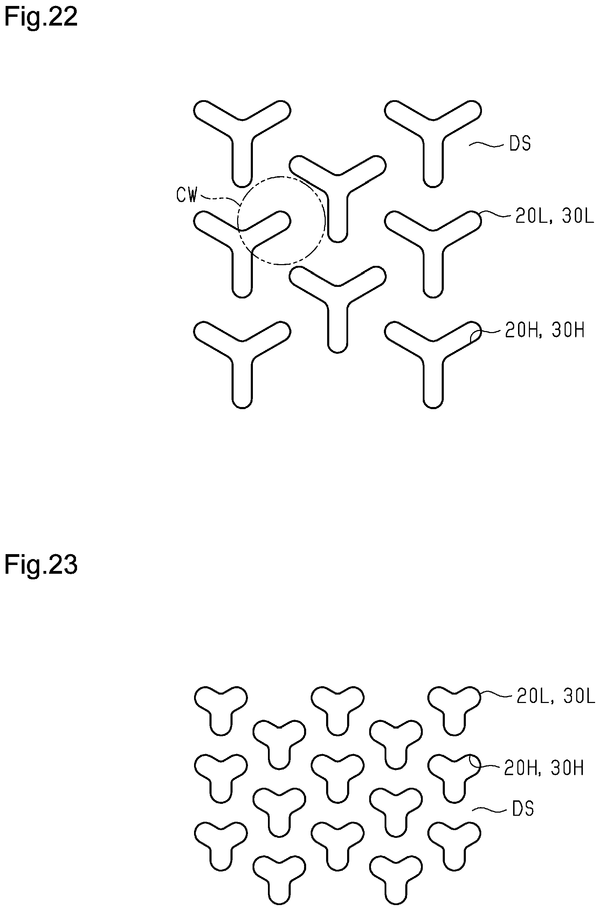

· As illustrated in Fig. 22, the openings of the upper through-holes 20H may have the shape of two or more line segments intersecting at one point. The lower through-hole 30H may also have the shape of two or more line segments intersecting at one point. The shape of two line segments intersecting at one point may be a T-shape, an X-shape, or a polygonal line shape. The shape of three or more line segments intersecting at one point may be a Y-shape or a radial shape. When two line segments intersecting at one point is referred to as one line segment pair, a shape including two or more line segment pairs may be an E-shape, an E-shape, an H-shape, a K-shape, or a polygonal line shape. As for an opening having the shape of two or more line segments intersecting at one point, a greater number of line segments intersecting at one point and a greater number of line segment pairs increase the length of the opening edges EA, EB in the unit area. This configuration allows the partition surface density to be independently increased while maintaining the opening sizes 2W, 2L and the inter-opening sizes 2WP, 2LP. As such, not only the formation period of the connective tissue body M and the accumulation period of somatic stem cells SC can be shortened, but also the shape that achieves an advantageous effect equivalent to (5) above has a greater degree of freedom.

When the upper through-holes 20H and the lower through-holes 30H define openings having a Y-shape or a radial shape, the through-holes 20H and 30H may be arranged as follows. That is, multiple through-holes 20H, 30H may be placed around a through-hole 20H, 30H such that the multiple through-holes 20H, 30H are set on an arrangement line CW, which is a circle that has its center at one end of the opening of the through-hole 20H, 30H and has a radius equal to the inter-opening size 2LP. In this case, the length of one line segment may be equal to the inter-opening size 2LP, which is the radius of the arrangement line CW, or may be longer than the inter-opening size 2LP.

· As illustrated in Fig. 23, when the openings of the through-holes 20H and 30H have the shape of two or more line segments intersecting at one point, the width of one line segment may be approximately equal to the length of the line segment. In this case, the length of one line segment may be equal to the inter-opening size 2LP, which is the radius of the arrangement line CW, or may be shorter than the inter-opening size 2LP.

As described above, the openings of the upper through-holes 20H and the openings of the lower through-holes 30H may have the shape of two or more line segments intersecting at one point. The opening size 2W, 2L of such openings is the diameter of the largest circle inscribed in the opening and touching the opening at two or more points. When the opening has a regular triangular shape, a square shape, a regular pentagonal shape, or a regular hexagonal shape, the opening size 2W, 2L of the opening is the length of one side of the regular polygon. When the opening has a flat shape, such as a rectangular shape or an elliptical shape, the opening size 2W, 2L of the opening is the width in the short axis direction.

### [Device Structure]

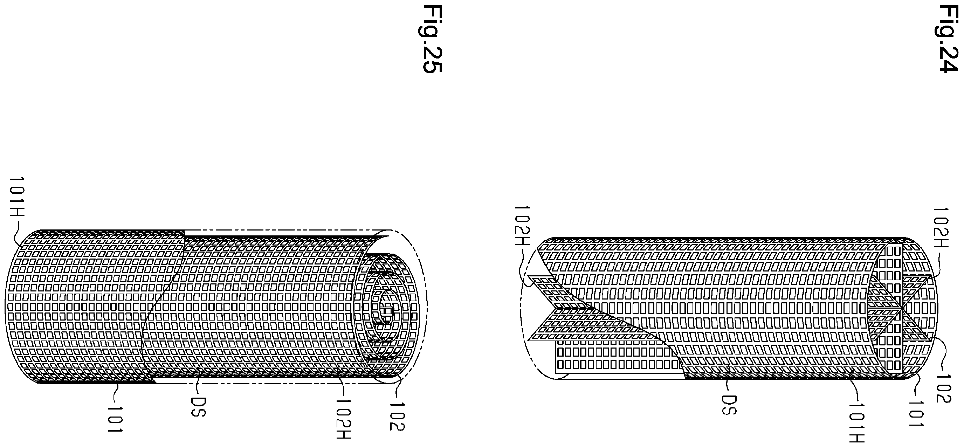

· As illustrated in Fig. 24, a tissue body formation device may include an exterior portion 101, which includes through-holes 101H, and an interior partition wall, which is located inside the exterior portion 101 and formed by interior metal plates covered with non-degradable resin coatings DS. In addition to the exterior portion 101, the interior partition wall may also include through-holes 102H.

For example, a tissue body formation device may include an interior portion 102 inside the exterior portion 101, which has the shape of a thin cylinder including through-holes 101H. The interior portion 102 may be formed by interior metal plates covered with non-degradable resin coatings DS and have the shape of thin plates including through-holes 102H. The interior portion 102 may have the shape of plates extending in the radial direction and the extending direction of the exterior portion 101. The interior portion 102 is fixed to opposite ends of the exterior portion 101 with the edges extending in the extending direction of the interior portion 102 separated from the inner circumference surface of the exterior portion 101. The interior portion 102 promotes the formation of dense connective tissue MH from the living tissue material entering the interior of the exterior portion 101 through the openings of the exterior portion 101. This increases the toughness of the connective tissue body M that requires to be thick and long, such as tendons and ligaments.

The planar interior portion 102 may be pulled out from the exterior portion 101 before the connective tissue body M is separated from the exterior portion 101, or may be pulled out from the exterior portion 101 after the connective tissue body M is separated from the exterior portion 101. The gap formed by pulling out the interior portion 102 is immediately filled by the adhesion of the connective tissue body M itself after the connective tissue body M is transplanted.

· As illustrated in Fig. 25, a tissue body formation device may include a cylindrical exterior portion 101 and an interior portion 102, which is formed by an interior metal sheet covered with a non-degradable resin coating DS. The interior portion 102 may have the shape of a single thin sheet including through-holes 102H. The interior portion 102 is wound in a spiral inside the exterior portion 101. The interior portion 102 promotes the formation of dense connective tissue MH from the living tissue material entering the interior of the exterior portion 101 through the openings of the exterior portion 101. By spreading the wound interior portion 102, it is possible to form a sheet-like connective tissue body M that requires to have a large area.

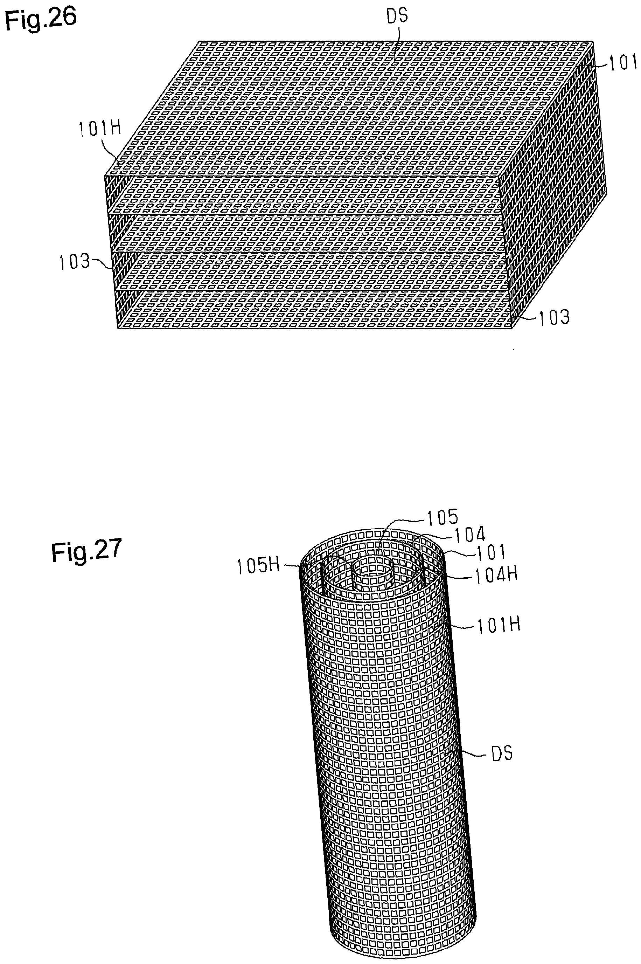

· As illustrated in Fig. 26, a tissue body formation device may be a laminate of exterior portions 101 stacked at intervals. Each exterior portion 101 may be formed by an exterior metal plate covered with a non-degradable resin coating DS and has the shape of a thin plate including through-holes 102H. The tissue body formation device may include positioning members 103, which position the exterior portions 101. Each positioning member 103 may also be formed by a metal plate covered with a non-degradable resin coating DS and have the shape of a thin plate including through-holes 102H. The openings in the exterior portion 101 of the uppermost layer and the openings in the exterior portion 101 of the lowermost layer facilitate the formation of the dense connective tissue MH from the living tissue material entering through these openings.

· As illustrated in Fig. 27, a tissue body formation device may include a multilayer tubular interior member, which is formed by metal sheets covered with non-degradable resin coatings DS and include through-holes 102H. For example, a tissue body formation device may include a first interior member 104 inside a cylindrical exterior portion 101. The first interior member 104 may be made of a metal sheet covered with a non-degradable resin coating DS, include through-holes 104H, and have the shape of a thin cylinder. The tissue body formation device may also include a second interior member 105 inside the first interior member 104. The second interior member 105 may be made from a metal sheet covered with the non-degradable resin coating DS and include through-holes 105H.

The first interior member 104 is fixed to opposite ends of the exterior portion 101 with the outer circumference surface of the first interior member 104 separated from the inner circumference surface of the exterior portion 101. The first interior member 104 promotes the formation of dense connective tissue MH from the living tissue material entering the interior of the exterior portion 101 through the openings of the exterior portion 101 and also the formation of dense connective tissue MH from the living tissue material entering through the openings of the first interior member 104.

The second interior member 105 is fixed to opposite ends of the exterior portion 101 with the outer circumference surface of the second interior member 105 separated from the inner circumference surface of the first interior member 104. The second interior member 105 promotes the formation of dense connective tissue MH from the living tissue material entering the inside of the first interior member 104 through the openings of the first interior member 104, and also the formation of dense connective tissue MH from the living tissue material entering through the openings of the second interior member 105.

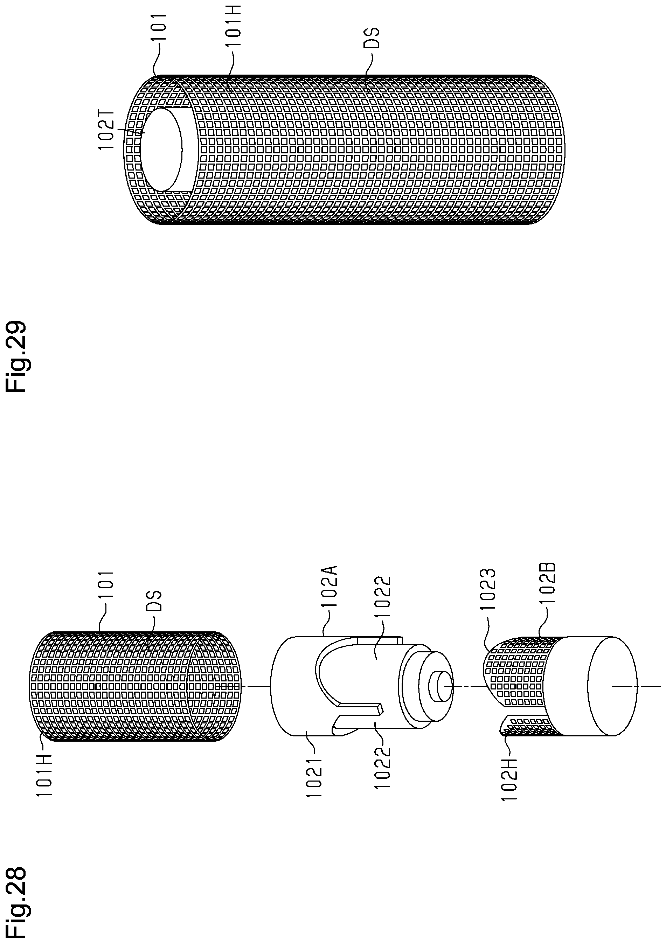

· As illustrated in Fig. 28, a tissue body formation device may also be embodied as a specialized device for forming a heart valve. The tissue body formation device includes a cylindrical exterior portion 101, a blood vessel interior member 102A housed inside the exterior portion 101, and a valve interior member 102B housed inside the exterior portion 101. The exterior portion 101 and the valve interior member 102B are made of metal sheets covered with non-degradable resin coatings DS and include through-holes 101H and 102H extending from the outside to the inside in the radial direction.

The blood vessel interior member 102A has a multi-stage columnar shape extending in the axial direction of the exterior portion 101. The blood vessel interior member 102A is spaced apart from the inner surface of the exterior portion 101 by the thickness of the blood vessel, which is an example of a connective tissue body M. The outer circumference surface of the blood vessel interior member 102A includes an upper outer circumference surface 1021, which is a cylindrical surface for forming only a blood vessel, and valve formation portions 1022, which are recessed sections recessed from the upper outer circumference surface 1021 by the thickness of the valve. The three valve formation portions 1022 are depressions evenly arranged in the circumferential direction of the blood vessel interior member 102A.

The valve interior member 102B has a cylindrical shape extending in the axial direction of the exterior portion 101. The valve interior member 102B is spaced apart from the inner surface of the exterior portion 101 by the thickness of the blood vessel. The valve interior member 102B includes three valve covering pieces 1023 arranged in the circumferential direction of the valve interior member 102B. Each valve covering piece 1023 is made of a metal sheet covered with a non-degradable resin coating DS, and includes through-holes 102H. The valve covering piece 1023 is spaced apart from the inner surface of the exterior portion 101 by the thickness of the blood vessel and spaced apart from the valve formation portion 1022 by the thickness of the valve. The valve covering pieces 1023 are placed inside the exterior portion 101 in a state in which they are fitted to the valve formation portions 1022.

The tissue body formation device forms a connective tissue body M between the exterior portion 101 and the blood vessel interior member 102A and between the exterior portion 101 and the interior valve interior member 102B. The connective tissue body M has the shape of a single cylinder and serves as a blood vessel. The tissue body formation device also forms a connective tissue body M that functions as a valve integral with the connective tissue body M for the blood vessel between the valve formation portion 1022 and the valve covering pieces 1023. In this manner, the layer thickness H1 of the dense connective tissue MH can be increased between the exterior portion 101 and the blood vessel interior member 102A and between the exterior portion 101 and the valve interior member 102B. Also, the layer thickness H1 of the dense connective tissue MH can be increased between the valve formation portion 1022 and the valve covering piece 1023. As a result, the inner lumen surface of the blood vessel, which is an example of a dense section, and the valve, which is an example of a dense section, can be formed as the connective tissue body M with increased toughness.

· As illustrated in Fig. 29, a tissue body formation device may include an exterior portion 101, which has the shape of a straight cylinder, and an interior portion 102T, which has the shape of a column extending in the axial direction of the exterior portion 101. That is, the shape of the tissue body formation device is not limited to a ring shape, and may be modified to a straight tube shape.

### [Non-degradable Resin Coating DS]

· The section of a connective tissue body M that includes dense connective tissue MH is a dense section, and the section that includes loose connective tissue ML is a loose section. The tissue body formation device may be configured such that the surface that is to be in contact with a dense section has a greater contact angle with water than the surface that is to be in contact with a loose section. Also, the tissue body formation device may be configured such that the surface that is to be in contact with a dense section has a lower compressive strength than the surface that is to be in contact with a loose section. In this case, the tissue body formation device satisfies [Condition 1] regarding the compressive strength of the surface in contact with the dense section, and satisfies [Condition 2] regarding the contact angle with water.

For example, when the connective tissue body M is used as a blood vessel, the higher density of the section of the connective tissue body M that is used as the inner lumen surface of the blood vessel limits the penetration of blood into the connective tissue body M. Accordingly, the connective tissue body M is expected to limit thrombus formation. On the other hand, the lower density of the section of the connective tissue body M that is used as the outer circumference surface of the blood vessel facilitates the infiltration by surrounding tissue in the living body. This is expected to promote engraftment. To this end, in Fig. 29, the surface of the interior portion 40, 102T for forming the inner lumen surface of the blood vessel satisfies [Condition 1] and [Condition 2]. In contrast, the surface of the exterior portion 10, 101 for forming the outer circumference surface of a blood vessel has a higher compressive strength and a lower contact angle with water than the surface of the interior portions 40, 102T.

For example, as described in the above modification, when the connective tissue body M is used as a heart valve, the high density of the section of the connective tissue M that is used as the outer surface of the valve limits the penetration of the blood into the connective tissue body M. As such, the connective tissue body M is expected to limit thrombus formation. To this end, in Fig. 28, the surfaces of the valve formation portions 1022 for forming the valve and the valve covering pieces 1023 satisfy [Condition 1] and [Condition 2]. Also, the surface of the blood vessel interior member 102A for forming the inner lumen surface of the blood vessel satisfies [Condition 1] and [Condition 2]. In contrast, the surface of the exterior portion 101 for forming the outer circumference surface of the blood vessel has a higher compressive strength and a lower contact angle with water than the surfaces of the valve formation portion 1022, the valve covering pieces 1023, and the blood vessel interior member 102A.

With this tissue body formation device, the surface of the tissue body formation device that is to be in contact with a loose section has a lower compressive strength than the surface of the tissue body formation device that is to be in contact with a dense section. Also, the surface of the tissue body formation device that is to be in contact with a loose section has a smaller contact angle with water than the surface of the tissue body formation device that is to be in contact with a dense section. This allows the dense connective tissue MH to be unevenly distributed in predetermined sections of the connective tissue body M that include dense connective tissue MH, and also allows the loose connective tissue ML to be unevenly distributed in predetermined sections of the connective tissue body M the include loose connective tissue ML.

· The thickness of the hollow space does not have to be uniform in the direction in which the hollow space extends. The thickness may gradually change from one end to the other in the direction in which the hollow space extends, or the hollow space may include a section in which the thickness gradually changes and a section in which the thickness is uniform.

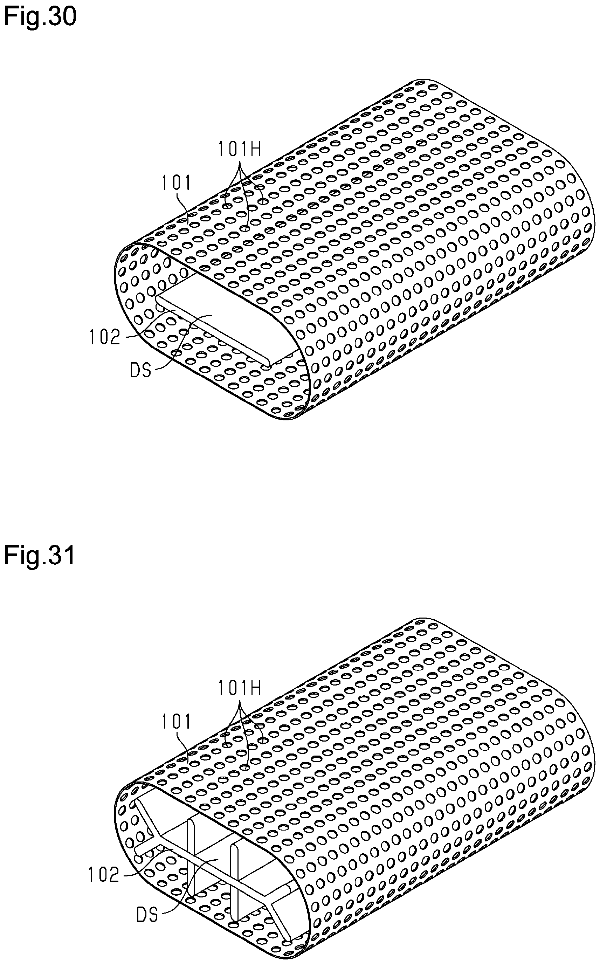

· When the connective tissue body M is used as a solid tissue body, such as a tendon or a ligament, the higher density inside the connective tissue body M is expected to strengthen the tendon or the human body. To this end, as illustrated in Fig. 30, the exterior portion 101 may have the shape of a flat elliptical ring extending in one direction, and the interior portion 102 may have the shape of a plate that is parallel to the direction in which the exterior portion 101 extends and made from a non-degradable resin. This configuration can include a greater number of through-holes facing the non-degradable resin than a configuration in which the interior portion 102 is columnar, thereby further increasing the strength of the connective tissue body M.

Furthermore, as illustrated in Fig. 31, the interior portion 102 may include a planar portion, which is made of a non-degradable resin and parallel to the direction in which the exterior portion 101 extends, and projection pieces projecting from the planar portion toward the exterior portion 101. In other words, the interior portion 102 may be a division wall that partitions the hollow space into multiple spaces that communicate with each other. This configuration can have the non-degradable resin of a larger surface area in the hollow space and also include a greater number of through-holes 101H facing the non-degradable resin, as compared to a configuration in which the interior portion 102 is a single plate, thereby further increasing the strength of the connective tissue body M.

### [Accumulation of Somatic Stem Cells SC]

· As described above, the technique of increasing the layer thickness H1 of dense connective tissue MH involves the technique of accumulating many collagen-producing fibroblasts in the hollow space, and also the technique of accumulating many somatic stem cells SC that become fibroblasts in the hollow space. When the tissue body formation device is used in a manner in which the tissue body formation device is removed from the biological environment MA before the entire hollow space is filled with a connective tissue body M, a greater number of somatic stem cells SC can be extracted in a short period of time using the pockets or voids in the connective tissue body M. The number of extracted somatic stem cells SC increases under conditions that sharply increase the layer thickness H1 of the dense connective tissue MH.

That is, the connective tissue body M formed by the tissue body formation device may have pockets in the sections facing the through-holes 20H, 30H, 101H, and 102H, and also have voids within the connective tissue body M. These pockets and voids may accommodate loose connective tissue and somatic stem cells SC. Also, the connective tissue body M formed by the tissue body formation device may include a covering tissue body MB4, which is dense connective tissue MH, loose connective tissue, which is located between the covering tissue body MB4 and the inner surface of the exterior portion 10, 101, and somatic stem cells SC included in the loose connective tissue.

In this case, the loose connective tissue ML in the connective tissue body M is an example of a loose section that includes loose connective tissue ML. The inner surface of the exterior portion 10, 101 that is to be in contact with loose connective tissue ML in the tissue body formation device is an example of a surface that is to be in contact with a loose section. The covering tissue body MB4 in the connective tissue body M is an example of a dense section that includes dense connective tissue MH. The interior surface 40S of the interior portion 40 that is in contact with the dense connective tissue MH in the tissue body formation device is an example of a surface that is to be in contact with a dense section. As described above, a configuration that includes non-degradable resin of a larger surface area in the hollow space facilitates the growth of the dense connective tissue MH in the hollow space, thereby causing a large number of somatic stem cells SC to accumulate in the hollow space. The configuration is therefore suitable for the accumulation of somatic stem cells SC. Somatic stem cells SC include mesenchymal stem cells and pluripotent stem cells that express at least one of pluripotent stem cell markers SSEA4 and SSEA3.

The technical concepts described below may be derived from the above modifications.

### [Clause]

A method for accumulating somatic stem cells, the method including:
using a tissue body formation device including an exterior portion including a through-hole and an interior portion located in a hollow space surrounded by the exterior portion to form a connective tissue body from a living tissue material in the hollow space in an environment in which the living tissue material is present; and
accumulating somatic stem cells in a depression that is disposed in an outer surface of the connective tissue body and faces an opening of the through-hole, wherein
the through-hole has an opening size of 0.02 mm or greater and 2.5 mm or less,
a surface of the interior portion includes a non-degradable resin,
the non-degradable resin has a compressive strength of 10 MPa or greater and 60 MPa or less, and
the non-degradable resin has a contact angle with water of 90° or greater.

### REFERENCE SIGNS LIST

DS...Non-Degradable Resin Coating; EA, EB...Opening Edge; M...Connective Tissue Body; MH...Dense Connective Tissue; ML...Loose Connective Tissue; 2L, 2SW, 2W...Opening Size; 10,101 ...Exterior Portion; 40, 102, 102T...Interior Portion; 40S...Surface; 20H, 30H, 101H, 102H, 104H, 105H...Through-Hole; 20SH, 30SH...Small Through-Hole

## Claims

1. A tissue body formation device for forming a connective tissue body in an environment in which a living tissue material is present, the tissue body formation device comprising:
a partition wall that defines a hollow space for forming the connective tissue body from the living tissue material, wherein
an exterior portion of the partition wall that separates the environment from the hollow space includes through-holes that extend between the environment and the hollow space, the through-holes have an opening size of 0.02 mm or greater and 2.5 mm or less,
a section of the connective tissue body that includes dense connective tissue is a dense section,
a surface of the partition wall that is in contact with the dense section includes non-degradable resin,
the non-degradable resin has a compressive strength of 10 MPa or greater and 60 MPa or less, and
the non-degradable resin has a contact angle with water of 90° or greater.

2. The tissue body formation device according to claim 1, wherein
a section of the connective tissue body that includes loose connective tissue is a loose section, and
a surface of the partition wall that is in contact with the loose section has a contact angle with water that is less than a contact angle with water of the surface of the partition wall that is in contact with the dense section.

3. The tissue body formation device according to claim 1 or 2, wherein
the exterior portion includes:
a first metal plate including a first inner surface and the through-holes; and
a second metal plate including a second inner surface and the through-holes,
the first metal plate is joined to the second metal plate in a separable manner such that the first inner surface and the second inner surface face each other,
the first inner surface is a curved recess surface that is recessed away from the second inner surface in the first metal plate, and has a shape that winds in a spiral in the first metal plate,
the second inner surface is a curved recess surface that is recessed away from the first inner surface in the second metal plate, and has a shape that winds in a spiral in the second metal plate,
the first inner surface and the second inner surface separate the single hollow space that winds in a spiral from the environment, and
the through-holes are located in and around the first inner surface in the first metal plate and also located in and around the second inner surface in the second metal plate.

4. The tissue body formation device according to claim 3, wherein
the partition wall has a spiral shape that conforms to the hollow space, and includes an interior portion that defines the hollow space in a space surrounded by the first inner surface and the second inner surface, and
the surface in contact with the dense section includes a surface of the interior portion.

5. The tissue body formation device according to claim 1, wherein
a length between opening edges of the through-holes that are mutually adjacent is 0.2 mm or greater,
a ratio of an opening area of all of the through-holes that are disposed in a unit area of an outer surface of the partition wall to the unit area of the outer surface of the partition wall is an opening occupancy rate,
a ratio of a length of the opening edges of all of the through-holes that are disposed in a unit area of an outer surface of the exterior portion to the unit area of the outer surface of the exterior portion is a partition surface density,
the opening occupancy rate is 4% or greater and 70% or less and
the partition surface density is 0.8/mm or greater.

6. The tissue body formation device according to any one of claims 1 to 5, wherein a shape of openings of the through-holes includes a shape of two or more line segments intersecting at one point.

7. The tissue body formation device according to any one of claims 1 to 6, wherein a shape of openings of the through-holes represents visually perceivable information.

8. A tissue body formation method for forming a connective tissue body in a tissue body formation device by implanting the tissue body formation device in an environment that is other than a human body and in which a living tissue material is present,
wherein the tissue body formation device is the tissue body formation device according to any one of claims 1 to 7.
